# FASCICULE DE BREVET EUROPEEN

(11) **EP 1 634 574 B1**
(45) Date de publication et mention de la délivrance du brevet: **28.05.2008**
(21) Numéro de dépôt: 05291832.3
(22) Date de dépôt: 05.09.2005
(51) Int. Cl.: A61K 8/49, A61Q 5/06, C09B 69/00

(54) **Nouveaux colorants directs noirs di-noyaux heteroaromatiques**
Neuartige schwarze direktziehende heteroaromatische Zweiringfarbstoffe
Novel black two ring heteroaromatic direct dyes

(30) Priorité: 08.09.2004 FR 0451987
(43) Date de publication de la demande: 15.03.2006
(73) Titulaire: L'ORÉAL, 75008 Paris (FR)
(72) Inventeur: Fadli, Aziz, 77500 Chelles (FR)
(74) Mandataire: Fevrier, Murielle Françoise E.

(56) Documents cités:
- EP-A- 1 275 367
- EP-A- 1 459 732

## Description

L'invention a pour objet de nouveaux colorants directs di-noyaux hétéro-aromatiques, les compositions tinctoriales contenant ces colorants ainsi que le procédé de teinture des fibres kératiniques les mettant en oeuvre. En particulier, l'invention a pour objet des colorants directs di-noyaux hétéro-aromatiques comprenant un noyau pyridinique.

Il est connu de teindre les fibres kératiniques et en particulier les cheveux humains, avec des compositions de teinture contenant des colorants directs. Les colorants classiques qui sont utilisés sont en particulier des colorants du type nitrés benzèniques, anthraquinoniques, nitropyridiniques, azoïques, azoïques cationiques, xanthéniques, acridiniques aziniques, triarylméthane benzéniques nitrés ou des colorants naturels.

Ces colorants qui sont des molécules colorées et colorantes ayant une affinité pour les fibres sont appliqués sur les fibres kératiniques pendant un temps nécessaire à l'obtention de la coloration désirée, puis rincés.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

La demande de brevet EP 1166754 décrit une composition tinctoriale qui comprend des colorants directs phénazinium azoiques cationiques. Du fait de la présence de la fonction azoïque, ces composés lorsqu'ils sont mis en oeuvre en présence d'un agent réducteur tel que l'acide érythorbique, métabisulfite ou du sulfite sont instables ce qui se traduit par une destruction du système chromophorique.

Le but de la présente invention est de fournir de nouveaux colorants directs qui ne présentent pas les inconvénients de la technique antérieure, en particulier des colorants directs permettant d'obtenir des nuances foncées, noires à grises, qui sont stables à la lumière, et résistants aux intempéries, aux lavages et à la transpiration, et en outre, stables dans un milieu classique de coloration.

En particulier, le but de la présente invention est de fournir des colorants directs noirs permettant de teindre les cheveux dans des nuances variant du gris au noir, sans avoir besoin d'éclaircir au préalable les cheveux, ainsi que des colorants directs noirs qui tout en s'affadissant ne changent pas de teinte, par exemple en virant après l'action d'un lavage, de lumière ou de sueur vers des nuances à reflets bleu, violet, rouge, vert, etc. Enfin, ces colorants directs noirs doivent permettre de conserver après plusieurs applications la nuance obtenue lors de la première application.

Ces buts sont atteints avec la présente invention qui a pour objet une composition tinctoriale comprenant, dans un milieu approprié, un composé de formule (I) suivante : 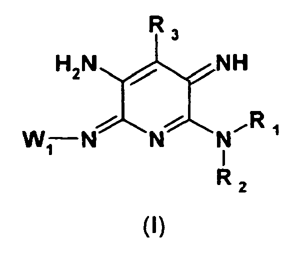 dans laquelle
- R₃ représente
   - un atome d'hydrogène,
   - une chaîne hydrocarbonée en C₁-C₁₀ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₃ ne comportant pas de liaison peroxyde, ni de radicaux diazo, nitro ou nitroso, R₃ n'étant pas relié au cycle pyridinique par un atome d'oxygène, d'azote ou de soufre ;
- R₁ et R₂ représentent indépendamment l'un de l'autre :
   - un atome d'hydrogène
   - une chaîne hydrocarbonée en C₁-C₁₀ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène; R₁ et R₂ ne comportant pas de liaison peroxyde, ni de radicaux diazo, nitro ou nitroso, et R₁ et R₂ n'étant pas directement reliés à l'atome d'azote par un atome d'oxygène, de soufre ou d'azote.
   - un radical cationique -D-Z dans lequel Z est un radical onium et D est une chaine hydrocarbonée en C₁-C₁₄ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂; le bras D n'étant pas relié à l'atome d'azote par un atome d'azote, d'oxygène ou de soufre, ou
- R₁ et R₂ forment ensemble avec l'atome d'azote auquel ils sont rattachés un cycle de formule (IV): 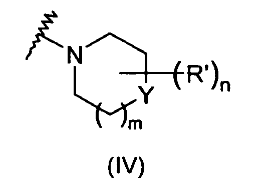 dans laquelle :
   - R' représente :
      - un atome d'halogène tel que fluor, chlore, brome ;
      - un radical alkyle en C₁-C₄ éventuellement substitué par un ou plusieurs radicaux choisis parmi les radicaux hydroxy, carboxy, alcoxycarbonyle en C₁-C₄, alkyl(C₁-C₄)amido (alkyl(C₁-C₄)CONH-), alkyl(C₁-C₄)carboxamido, (alkyl(C₁-C₄) NHCO-), alkyl(C₁-C₄)sulfonyle (alkyl(C₁-C₄) SO₂-), alcoxy en C₁-C₄, alkyl(C₁-C₄)SUifonamido ( alkyl(C₁-C₄)SO₂NH-), alkyl(C₁-C₄)sulfamoyle (alkyl(C₁-C₄) NH SO₂-);
      - NR'₃R'₄ avec R'₃ et R'₄, identiques ou différents, représentent, un atome d'hydrogène ; un radical alkyle en C₁-C₄ éventuellement substitué par un ou plusieurs radicaux choisis parmi les radicaux hydroxy, alcoxy en C₁-C₄, amino, mono ou di alkylamino, alkyl(C₁-C₄)CO-, alkyl(C₁-C₄) NHCO-, alkyl(C₁-C₄)SO₂,
      - un radical carboxy ;
      - un radical alcoxycarbonyle en C₁-C₄ ;
      - un radical alkyl(C₁-C₄)amido (alkyl(C₁-C₄)CONH-) ;
      - un radical alkyl(C₁-C₄)sulfonyle (alkylSO₂-) ;
      - un radical alkylsulfonamido (alkyl(C₁-C₄) SO₂NH -) ;
      - un radical hydroxy ;
      - un radical alcoxy en C₁-C₄ ;
      - un radical hydroxyalcoxy en C₂-C₄ ;
      - un radical alkyl(C1-C4)carboxamido (alkyl(C1-C4) NHCO-) ;
      - un radical alkyl(C₁-C₄)sulfamoyle (alkyl(C₁-C₄)-NH-SO₂-) :
      - un radical thioéther en C₁-C₄ ;
      - un radical sulfonique (SO₃H) pouvant être sous forme de sel ;
      - un radical cationique -D1-Z dans lequel D1 est une liaison covalente ou D,

   - n est un nombre entier compris entre 0 et 12,
   - m est un nombre entier compris entre 0 et 2,
   - Y représente un atome de carbone, un atome d'oxygène, un radical NR'₅ ou un radical N⁺R'₆R'₇ avec R'₅ a la même signification que R'₃ ;R'₆ et R'₇ ont la même signification que R'₃ et sont différents d'un atome d'hydrogène,
   étant entendu que lorsque n > 1, les radicaux R' peuvent être différents les uns des autres,
- W₁ représente un radical hétérocycle aromatique choisi parmi les radicaux suivants 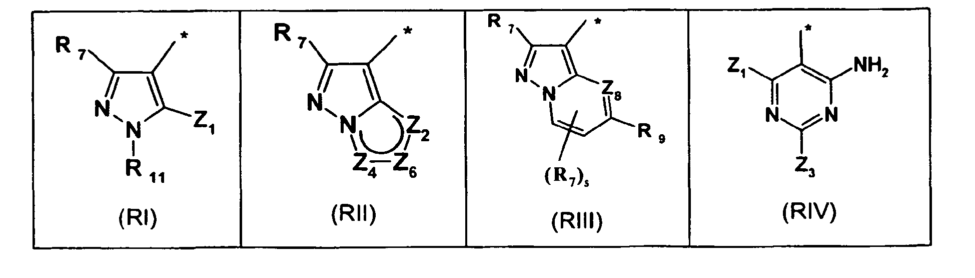 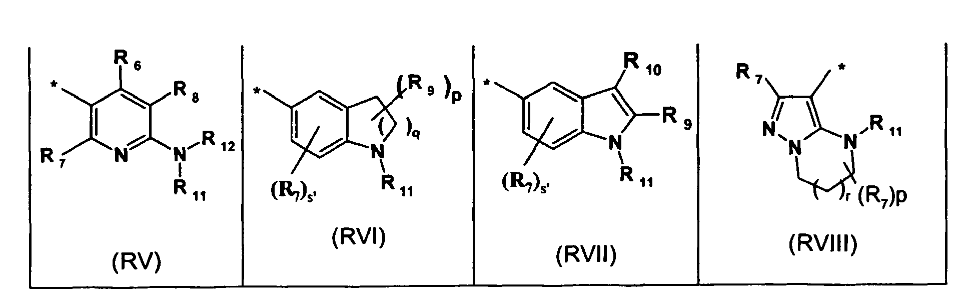 dans lesquels
   - s est 0, 1 ou 2 ;
   - s' est 0, 1, 2 ou 3,
   - Z₁ et Z₃, représentent indépendamment les uns des autres un radical hydroxy ou NR₁₁R₁₂
   - Z₂, Z₄, Z₆ représentent indépendamment les uns des autres un atome d'azote, un radical CR₁₂ ou NR₁₁ sous réserve qu'au moins l'un d'entre eux représente un radical CR₁₂ et qu'il n'y est pas plus de 3 atomes d'azote contigus,
   - Z₈ représente un atome d'azote, un radical CR₁₂,
   - R₆ et R₈ indépendamment l'un de l'autre ont les mêmes significations que R₃.
   - R₇, R₉, R₁₀, R₁₁ et R₁₂ représentent indépendamment les uns des autres
      - un atome d'hydrogène,
      - une chaîne hydrocarbonée en C₁-C₁₀ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; les radicaux R₇ et R₉ à R₁₂ ne comportant pas de liaison peroxyde, ni de radicaux diazo ou nitroso et les radicaux R₁₁ et R₁₂ n'étant pas reliés directement à l'atome d'azote par un atome d'oxygène, de soufre ou d'azote,
      étant entendu que les radicaux R₇ et R₉ peuvent être indépendants l'un de l'autre,
      - p peut prendre les valeurs 4 à 8,
      - q peut prendre les valeurs 1 à 3,
      - r peut prendre les valeurs 0 à 2,
      - * indique le point d'attache de W1 dans la formule (I),
      étant entendu que le composé de formule (I) ne peut comporter plus d'un radical cationique D1-Z.

La présente invention a aussi pour objet une composition tinctoriale contenant dans un milieu approprié au moins un colorant direct de la présente invention. La composition de la présente invention est particulièrement utile pour la teinture des fibres kératiniques, en particulier les fibres kératiniques humaines.

Lorsque dans la définition de Z₂, Z₄, Z₆ qui représentent indépendamment les uns des autres un atome d'azote, un radical CR₁₂ ou NR₁₁, il est précisé qu'il ne peut y avoir plus de 3 atomes d'azote contigus, cela signifie qu'il n'est pas possible par exemple d'obtenir la structure suivante

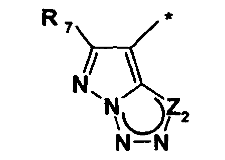

De plus, sauf indication contraire, les bornes délimitant l'étendue d'une plage de valeurs sont comprises dans cette plage de valeurs.

Dans le cadre de la présente invention, les composés de formule (I) sont non seulement ceux décrits par la formule (I) mais toute autre forme tautomérique telle que par exemple la forme tautomérique suivante :

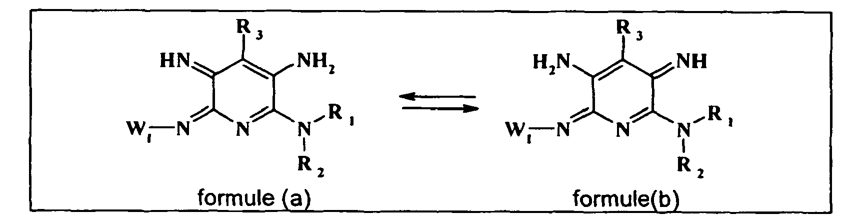

Dans les exemples qui sont donnés dans la suite de la description, une seule de ces formes tautomériques sera indiquée.

Dans la formule (I), R₃ est de préférence choisis choisi parmi un atome d'hydrogène ou un radical alkyle en C₁-C₄ éventuellement substitué par un ou plusieurs radicaux choisis parmi les radicaux hydroxy, alcoxy en C₁-C₂, amino, (mono)- ou (di)alkylamino en C₁-C₂. A titre d'exemple, R₃ représente un atome d'hydrogène, un radical méthyle, éthyle, 2-hydroxyéthyle, de préférence un atome d'hydrogène ou un radical méthyle.

Les radicaux R₁ et R₂ sont de préférence indépendamment choisis parmi l'hydrogène, un radical alkyle en C₁-C₆ éventuellement substitué par un hydroxy, un alkoxy, un amino, un (mono)- ou (di)alkylamino en C₁-C₄, un radical -D-Z avec Z pouvant être un imidazolium, un trialkyl(C₁-C₄)ammonium, un pyridinium, un pipéridinium. A titre d'exemple R₁ et R₂ sont choisis parmi un radical hydrogène, méthyle, éthyle, hydroxyéthyle, propyle, propylimidazolium, propyltriméthylammonium.

Lorsque R₁ et R₂, forment avec l'atome d'azote auquels ils sont rattachés un hétérocycle de 5 à 8 chaînons de formule (IV), cet hétérocycle est de préférence choisi parmi les hétérocycles pyrrolidine, pipéridine, homopipéridine, pipérazine, homopipérazine, diazépane. A titre d'exemple, l'hétérocycle est choisi parmi la pyrrolidine, la 3-hydroxypyrrolidine, la 3-aminopyrrolidine, la 3-N,N-diméthylaminopyrrolidine, la 3-acétamidopyrrolidine, la 3-(méthylsulfonylamino)-pyrrolidine, la proline, la 3-hydroxyproline, le 3-(N-methylimidazolium)-pyrrolidine, le 3-(trialkyl(C1-C4)ammonium)pyrrolidine, la pipéridine, l'hydroxypipéridine, l'homopipéridine, le diazépane, N-méthyl homopipérazine, la N β-hydroxyéthylhomopipérazine et leurs sels d'addition. De préférence, R₁ et R₂ forment avec l'atome d'azote auquels ils sont rattachés un cycle pyrrolidinique éventuellement substitué. Selon un mode de réalisation, R' est de préférence un radical -D1-Z choisi parmi un imidazolium ou un trialkylammonium.

Les radicaux R'₅, R'₆ et R'₇ sont de préférence un alkyle en C₁-C₄.

Les radicaux R'₃ et R'₄ sont de préférence un alkyl(C₂-C₄)imidazolium, un alkyl(C₂-C₄)pyridinium, alkyl(C₂-C₄)trialkyl(C₂-C₄)ammonium.

Le radical cationique -D-Z peut être représenté par la formule (V) suivante 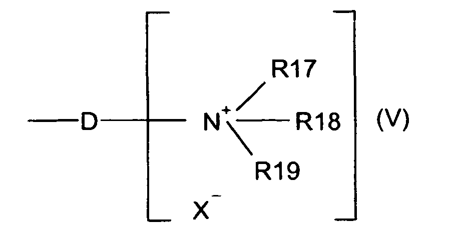 dans laquelle
- D est tel que défini précédemment,
- R₁₇, R₁₈ et R₁₉, pris séparément, représentent un radical alkyle en C₁-C₁₅; un radical monohydroxyalkyle en C₁-C₆; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, acylamino ou alkyl(C₁-C₆)sulfonyle ;
- R₁₇, R₁₈ et R₁₉ ensemble, deux à deux, peuvent conjointement former, avec l'atome d'azote quaternisé auxquels ils sont rattachés, un cycle saturé carboné à 5, 6 ou 7 chaînons pouvant contenir un ou plusieurs hétéroatomes, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkyl(C₁-C₆)carbonyle, un radical thio, un radical thioalkyle en C₁-C₆, un radical alkyl(C₁-C₆)thio, un radical amino, un radical amino mono- ou di-substitué par un radical alkyl(C₁-C₆), alkyl(C₁-C₆)carbonyle, acylamino ou alkyl(C₁-C₆)sulfonyle;
- l'un des R17, R18 ou R19 peut être relié à un des atomes de carbone ou d'azote du bras D pour former un cycle de 5 à 7 chainons, et
- X- est un contre ion.

X- représente en général un anion organique ou minéral par exemple choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁₋C₆)sulfonate tel que méthylsulfonate ; un arylsulfonate substitué ou non substitué par un radical alkyle en C₁-C₄ tel que par exemple un 4-toluylsulfonate. X- est de préférence un chlorure ; un méthylsulfate.

Selon une première variante de la formule (V), R₁₇, R₁₈ et R₁₉ séparément sont de préférence choisis parmi un radical alkyle en C₁-C₆, un radical monohydroxyalkyle en C₁-C₄, un radical polyhydroxyalkyle en C₂-C₄, un radical alcoxy(C₂-C₆)alkyle en C₁-C₄, un radical amidoalkyle en C₂-C₆, un radical trialkyl(C₁-C₆)silanealkyle en C₁-C₆.

Selon un deuxième variante de la formule (V), R₁₇ avec R₁₈ forment ensemble un cycle pyrrolidinium, pipéridinium, homopipéridinium, pipérazinium, homopipérazinium, morpholinium, R₁₉ est alors choisi parmi un radical alkyle en C₁-C₆ ; un radical monohydroxyalkyle en C₁-C₆ ; un radical polyhydroxyalkyle en C₂-C₆; un radical aminoalkyle en C₂-C₆, un radical aminoalkyle mono- ou di-substitué par un radical alkyl(C₁-C₆), alkyl(C₁-C₆)carbonyle; un radical carbamylalkyle en C₂-C₆ ; un radical trialkyl(C₁-C₆)silanealkyle en C₁-C₆ un radical alkyl(C₁-C₆)carboxyalkyle en C₁-C₆ ; un radical alkyl(C₁-C₆)carbonylaikyle en C₂-C₆ ;un radical N-alkyl(C₁-C₆)carbamylalkyle en C₂-C₆.

Selon un mode de réalisation préférée, R₁₇, R₁₈ et R₁₉ sont des radicaux alkyles.

Dans la formule (V), D est de préférence une chaîne alkylène en C₁-C₆ pouvant être substituée .

Le radical cationique -D-Z peut aussi être représenté par la formule (VI) 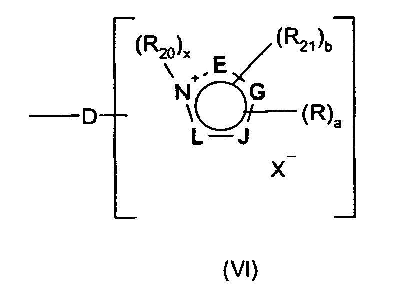 dans laquelle
- D est tel que défini précédemment,
- les sommets E, G, J, L, identiques ou différents, représentent un atome de carbone, d'oxygène, de soufre ou d'azote pour former un cycle pyrazolium, imidazolium, triazolium, oxazolium, isooxazolium, thiazolium, isothiazolium,
- a est un nombre entier compris entre 1 et 3 inclus ;
- b est 0 ou 1 ;
- R, identiques ou différents, représentent un atome d'hydrogène, d'halogène, un radical alkyle en C₁-C₆, un radical monohydroxyalkyle en C₁-C₆, un radical polyhydroxyalkyle en C₂-C₆, un radical alcoxy en C₁-C₆, un radical trialkyl(C₁-C₆)silanealkyle en C₁-C₆, un radical amido, un radical carboxyle, un radical alkylcarbonyle en C₁-C₆, un radical thioalkyle en C₁-C₆, un radical alkyl(C₁-C₆)thio, un radical amino di substituée par un radical alkyl(C₁-C₆), alkyl(C₁-C₆)carbonyle, alkyl(C₁-C₆)sulfonyle, un radical benzyle, un radical phényle éventuellement substitué par un ou plusieurs radicaux choisis parmi les radicaux méthyle, hydroxy, amino, methoxy étant entendu que les radicaux R sont portés par un atome de carbone,
- R₂₁ représente un radical alkyle en C₁-C₆; un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₂-C₆, un radical alkyl(C₁-C₆)carboxyalkyle en C₁-C₆, un radical benzyle ; étant entendu que le radical R₂₁ est porté par un atome d'azote,
- R₂₀ représente un radical alkyle en C₁-C₆ ; un radical monohydroxyalkyle en C₁-C₆ ; un radical polyhydroxyalkyle en C₂-C₆ ; un radical benzyle ; un radical aminoalkyle en C₁-C₆ un radical aminoalkyle en C₁-C₆ dont l'amine est mono-
ou di-subsitué par un radical alkyl(C₁-C₆), alkyl(C₁-C₆)carbonyle, acylamino ou alkyl(C₁-C₆)sulfonyle ; un radical carboxyalkyle en C₁-C₆ ; un radical carbamylalkyle en C₁-C₆ ; un radical trifluoroalkyle en C₂-C₆ un radical trialkyl(C₁-C₆)silanealkyle en C₁-C₆ un radical sulfonamidoalkyle en C₂-C₆ ; un radical alkyl(C₁-C₆)carboxyalkyle en C₁-C₆ un radical alkyl(C₁-C₆)sulfinylalkyle en C₂-C₆ ; un radical alkyl(C₁-C₆)sulfonylalkyle en C₂-C₆ ; un radical alkyl(C₁-C₆)carbonylalkyle en C₂-C₆; un radical N-alkyl(C₁-C₆)carbamylalkyle en C₂-C₆ ; un radical N-alkyl(C₁-C₆)sulfonamidoalkyle en C₂-C₆;
- x est égal à 0 ou 1 ;
   - lorsque x = 0, le bras de liaison D est rattaché à l'atome d'ammonium,
   - lorsque x = 1, le bras de liaison D est rattaché à l'un des sommets E, G, J ou L;
- X- est un contre-ion tel que défini précédemment.

De préférence, les sommets E, G, J et L forment un cycle imidazolium, pyrazolium, oxazolium, thiazolium et triazolium.

Selon un mode de réalisation particulier de la formule (VI), x est égal à 0 et D est une chaîne alkylène en C₁-C₄ pouvant être substituée.

Le radical cationique -D-Z peut aussi être représenté par la formule (VII) 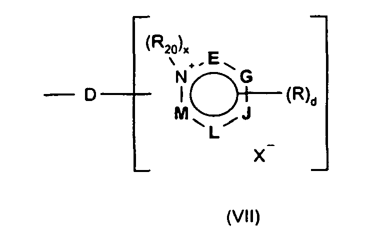 dans laquelle :
- D, R, R₂₀ sont tels que définis précédemment,
- les sommets E, G, J, L et M identiques ou différents, représentent un atome de carbone ou d'azote et forment un cycle choisi parmi les cycles pyridinium, pyrimidinium, pyrazinium et pyridazinium.
- d est un nombre entier compris entre 1 et 5 inclus ;
- x est égal à 0 ou 1
   - lorsque x = 0, le bras de liaison D est rattaché à l'atome d'ammonium,
   - lorsque x = 1, le bras de liaison D est rattaché à l'un des sommets E, G, J, Lou M;
- X- représente un contre ion tel que défini précédemment.

De préférence, les sommets E,G,J,L et M avec l'azote quaternisé du cycle forment un cycle choisi parmi les cycles pyridinium, pyrimidinium, pyrazinium et pyridazinium.

Selon une variante de la formule (VII), x est égal à 0 et R est choisi parmi un atome d'hydrogène, un radical alkyle en C₁-C₆, un radical monohydroxyalkyle en C₁-C₆, un radical polyhydroxyalkyle en C₂-C₆, un radical alcoxy en C₁-C₆, un radical alkylcarbonyle en C₁-C₆, un radical amino di-substitué par un radical alkyl(C₁-C₆), alkyl(C₁-C₆)carbonyle, alkyl(C₁-C₆)sulfonyle ; étant entendu que les radicaux R sont portés par un atome de carbone.

Selon une autre variante de la formule (VII), x est égal à 1, R₂₀ est choisi parmi un radical alkyle en C₁-C₆ un radical monohydroxyalkyle en C₁-C₆ un radical polyhydroxyalkyle en C₂-C₆ ; un radical aminoalkyle en C₂-C₆, un radical aminoalkyle en C₂-C₆ dont l'amine est mono- ou di- substituée par un radical alkyl(C₁-C₆), alkyl(C₁-C₆)carbonyle, un radical acylamino ou un radical alkyl(C₁-C₆)sulfonyle ; un radical carbamylalkyle en C₁-C₆ ; un radical alkyl(C₁-C₆)carbonylalkyle en C₁-C₆ ; un radical N-alkyl(C₁-C₆)carbamylalkyle en C₁-C₆; R est choisi parmi un radical alkyle en C₁-C₆, un radical monohydroxyalkyle en C₁-C₆, un radical polyhydroxyalkyle en C₂-C₆, un radical alcoxy en C₁-C₆, un radical alkylcarbonyle en C₁-C₆, un radical amino di- substitué par un radical alkyl(C₁-C₆), alkyl(C₁-C₆)carbonyle, alkyl(C₁-C₆)sulfonyle.

Dans un mode de réalisation préférée, R₂₀ est un radical alkyle en C₁-C₄ pouvant être substitué par un radical hydroxy ou méthoxy et R un radical hydrogène, un radical alkyle en C₁-C₄ pouvant être substitué éventuellement par un radical hydroxy ou méthoxy.

Dans la formule (I), W₁ représente en particulier les radicaux 5-aminopyrazole, 5-hydroxypyrazole, pyrazolo-[1,5-a]-pyridine, pyrazolo-[1,5-a]-pyrimidine, triaminopyrimidine et 2,3-diamino-6-alcoxy-pyridine.

Dans le cas où W₁ est choisi parmi les radicaux 5-aminopyrazole et 5-hydroxypyrazole de formule (RI), alors R₇ et R₁₁ sont indépendamment choisis parmi un atome d'hydrogène ; une chaîne hydrocarbonée en C₁-C₄ linéaire ou ramifiée, pouvant former un ou cycles carbonés comportant de 5 à 6 chaînons, et pouvant être saturées ou insaturées, les atomes de carbone peuvent être, indépendamment les uns des autres, substitués par un ou plusieurs atomes d'halogène ou radicaux hydroxy, amino; un radical alcoxy en C1-C2; un radical amino; un radical (di)alkyl(C₁-C₄)amino.

Selon un autre mode de réalisation particulier, W₁ représente un radical de formule (RIII): 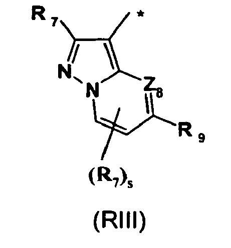 R₇, R₉ et Z₈ étant tels que définis précédemment.

Dans ce cas particulier, lorsque Z₈ représente CR₁₂ alors R₇, R₉ et R₁₂ identiques ou différents sont de préférence choisis parmi :
- un atome d'hydrogène,
- un radical amino ;
- une chaîne hydrocarbonée en C₁-C₈ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; les radicaux ne comportant pas de liaison peroxyde, ni de radicaux diazo ou nitroso et n'étant pas relié directement à l'atome d'azote par un atome d'oxygène, de soufre ou d'azote.

Dans ce mode de réalisation, les radicaux R₇, R₉ et R₁₂ sont de préférence choisis parmi un atome d'hydrogène ; un radical amino ; une chaîne hydrocarbonée en C₁-C₄ linéaire ou ramifiée et pouvant être saturées ou insaturées, les atomes de carbone peuvent être, indépendamment les uns des autres, substitués par un ou plusieurs atomes d'halogène ou radicaux hydroxy, amino ; un radical alcoxy en C1-C2 ; un radical amino ; un radical (di)alkyl(C₁-C₄)amino, éventuellement substitué.

Dans la formule (RIII), lorsque Z8 représente N alors R₇ et R₉ sont choisis parmi un atome d'hydrogène ; un radical alkyle en C₁-C₆ linéaire ou ramifiée ; un radical monohydroxyalkyle en C₁-C₆ un radical polyhydroxyalkyle en C₂-C₆ ; un radical aminoalkyle en C₁-C₆, un radical aminoalkyle en C₁-C₆ dont l'amine est mono- ou di-substituée par un radical alkyl(C₁-C₆), alkyl(C₁-C₆)carbonyle; un radical hydroxy ou amino, l'amino pouvant être éventuellement substitué par un ou plusieurs radicaux alkyles en C₁-C₁₀ linéaire ou ramifiée, pouvant former un ou plusieurs cycles carbonés comportant de 5 à 6 chaînons, et pouvant être saturées ou insaturées, les atomes de carbone peuvent être, indépendamment les uns des autres, substitués par un ou plusieurs atomes d'halogène ou radicaux hydroxy, amino ; un radical alcoxy en C1-C2.

Selon un mode de réalisation particulièrement préféré, W₁ est un dérivé pyrazolo-[1,5-a]-pyridine ou pyrazolo-[1,5-a]-pyrimidine dans lequel R₇, R₉ et R₁₂ sont choisis parmi l'hydrogène, un radical alkyle en C₁-C₄, un radical amino, un radical mono ou di alkylamino en C₁-C₄, un radical hydroxyalkyle en C₁-C₄; un radical alkoxy en C₁-C₄.

A titre d'exemple, les composés de formule (I) peuvent être cités :

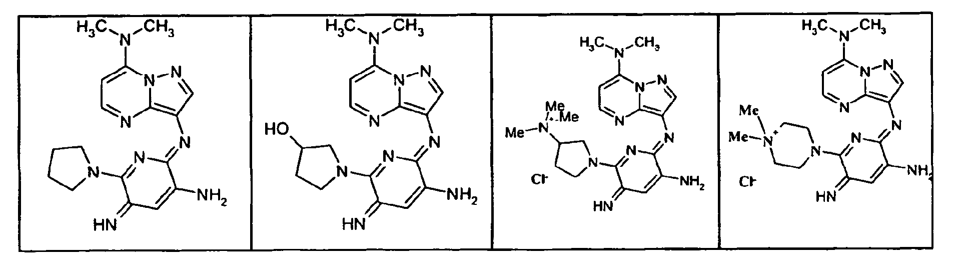

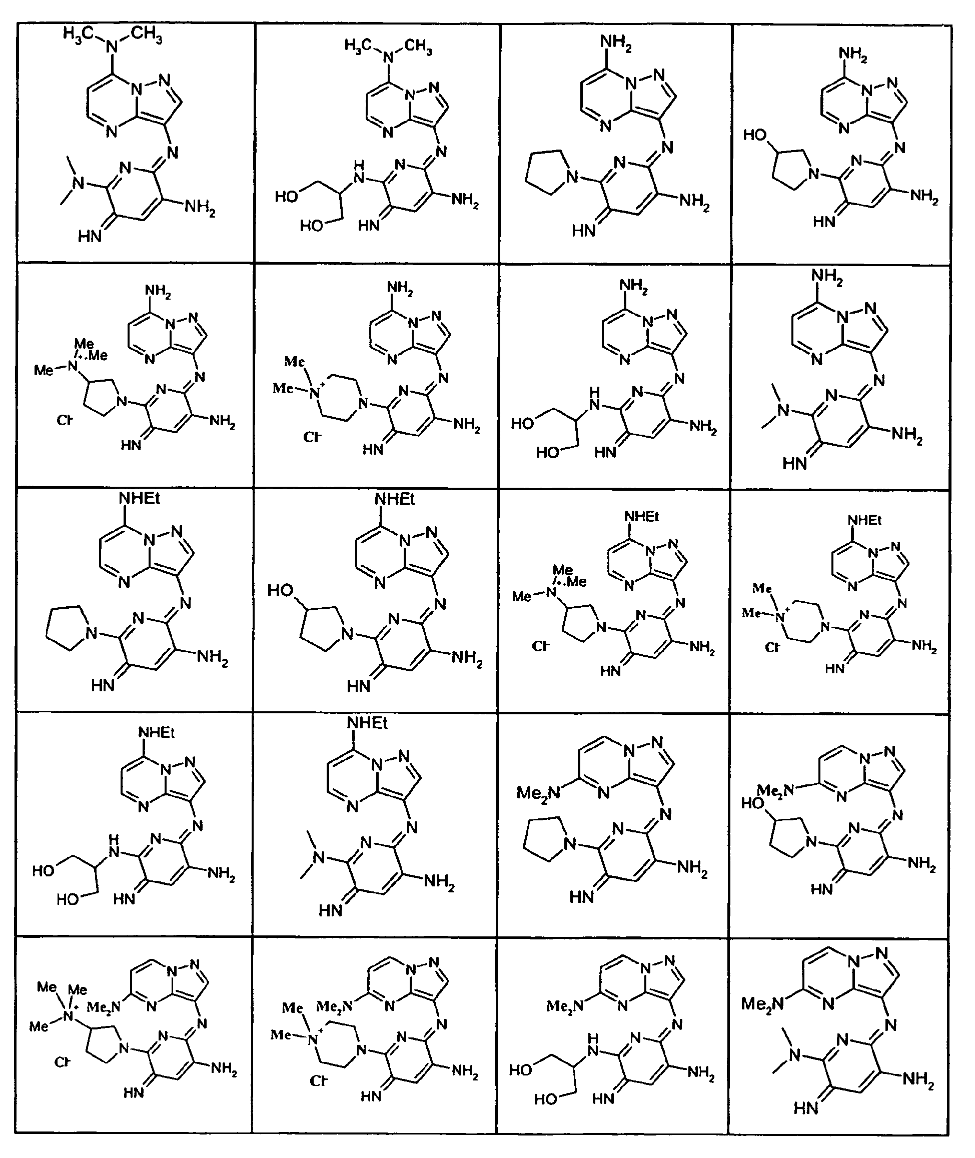

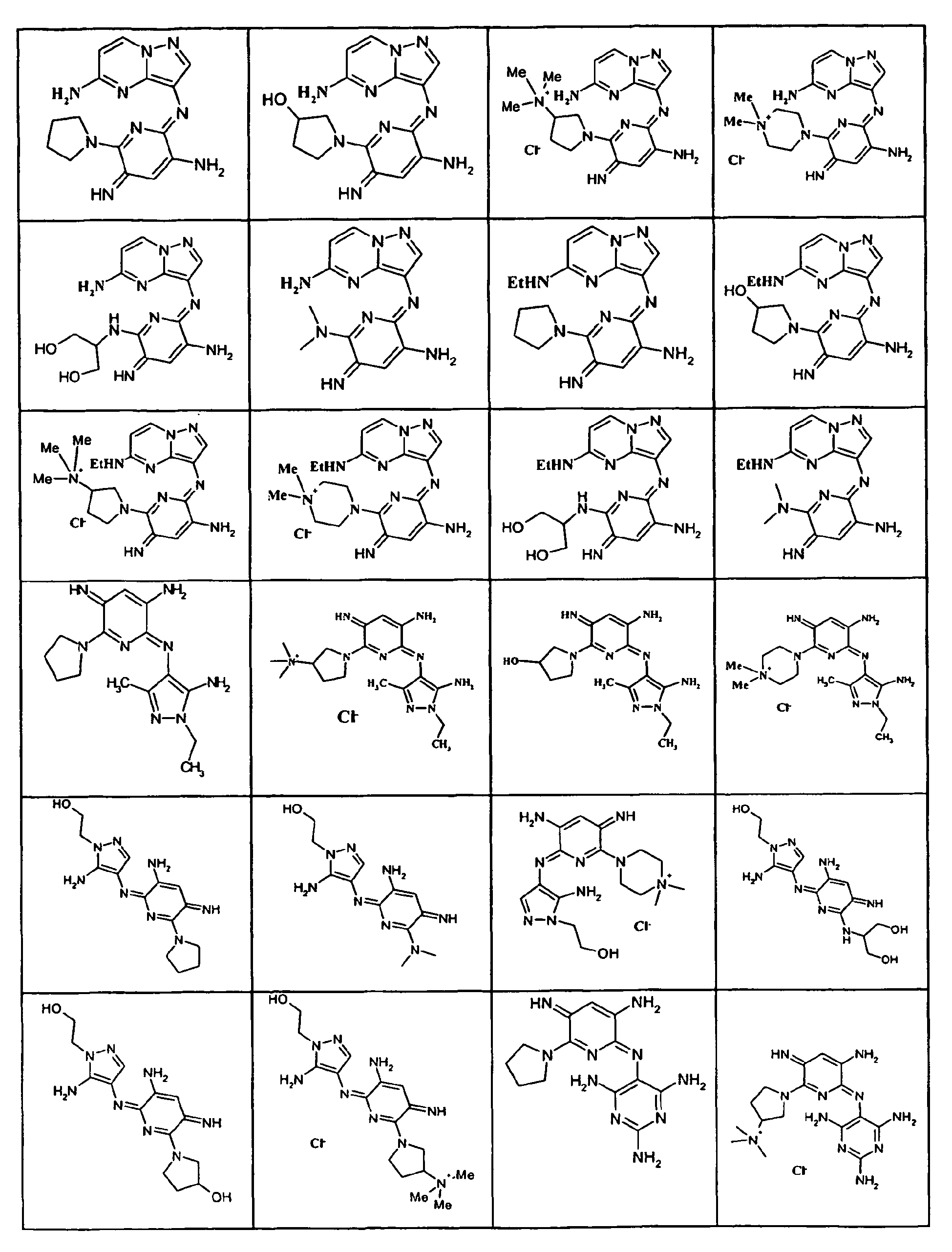

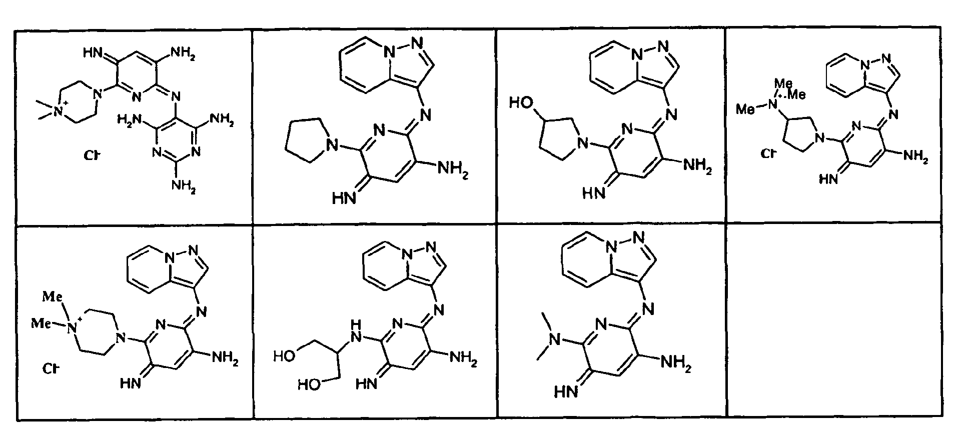

Selon un mode de réalisation préféré, le composé de formule (I) peut être représenté par une des formules suivantes : 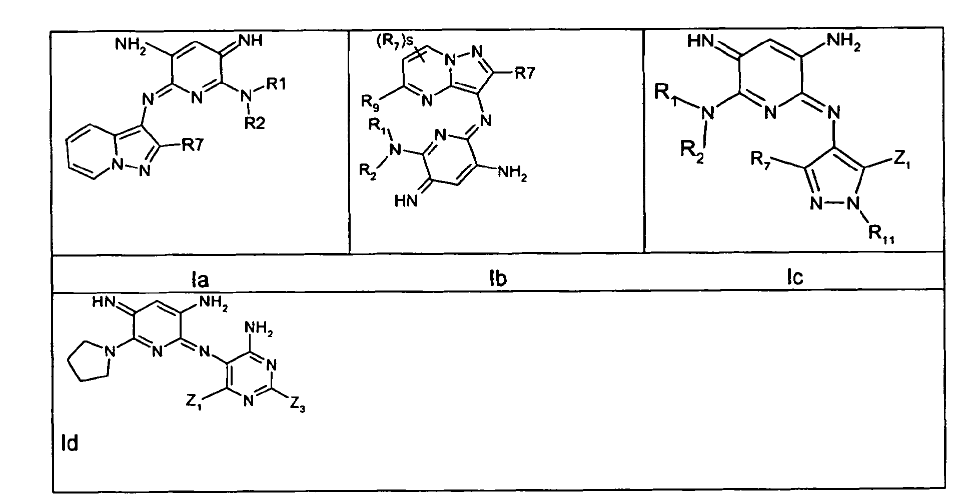 dans lesquelles R₁, R₂, R₆, R₇, R₈, R₉, R₁₁, Z₁, Z₂ et Z₃ sont tels que définis précédemment.

La composition de l'invention peut contenir une quantité de composé (1) variant de 0,005 à 10 % en poids du poids total de la composition. De préférence, une quantité de composé (1) est comprise entre 0,01 et 10 % en poids, de préférence de 0,1 à 5 %.

La composition tinctoriale conforme à l'invention peut en outre contenir des colorants directs différents des composés de formule (I). Ces colorants directs utiles selon l'invention sont par exemple choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques, les colorants directs de type tétraazapentaméthine et les colorants directs naturels.

Parmi les colorants directs benzéniques, on peut citer de manière non limitative les composés suivants:
- 1,4-diamino-2-nitrobenzène
- 1-amino-2 nitro-4-β- hydroxyéthylaminobenzène
- 1-amino-2 nitro-4-bis(β-hydroxyéthylfaminobenzène
- 1,4-Bis(β-hydroxyéthylamino)-2-nitrobenzène
- 1-β-hydroxyéthytamino-2-nitro-4-bis-(p-hydroxyéthytamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-f3-hydroxyéthyloxy-2-f3-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylam ino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants:
- chlorure de 1,3-diméthyl-2-[[4-(diméthylamino)phényl]azo]-1 H-Imidazolium,
- chlorure de 1 ,3-diméthyl-2-[(4-aminophényl)azo]-1 H-lmidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
Disperse Red 17, Acid Yellow 9, Acid Black 1, Basic Red 22, Basic Red 76, -Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl- 1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques, on peut citer les composés suivants :
- 2-β-hydroxyéthlyamino-5-[bis-β-4'-hydroxyéthyl)amino]anilino-1 ,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants de type tétraazapentaméthiniques utilisables selon l'invention, on peut citer les composés suivants figurant dans le tableau ci-dessous, An représentant en général un anion organique ou minéral par exemple choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que méthylsulfonate ; un arylsulfonate substitué ou non substitué par un radical alkyle en C₁-C₄ tel que par exemple un 4-toluylsulfonate. An est de préférence un chlorure ; un méthylsulfate.

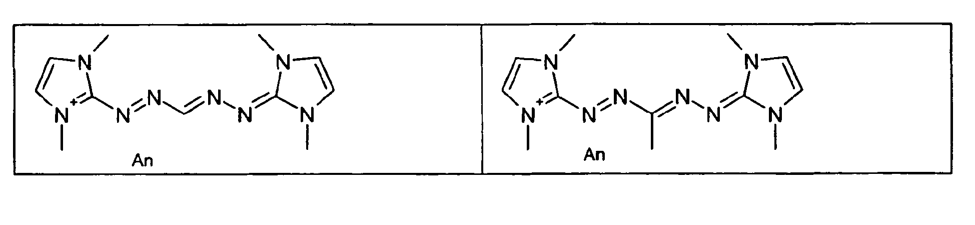

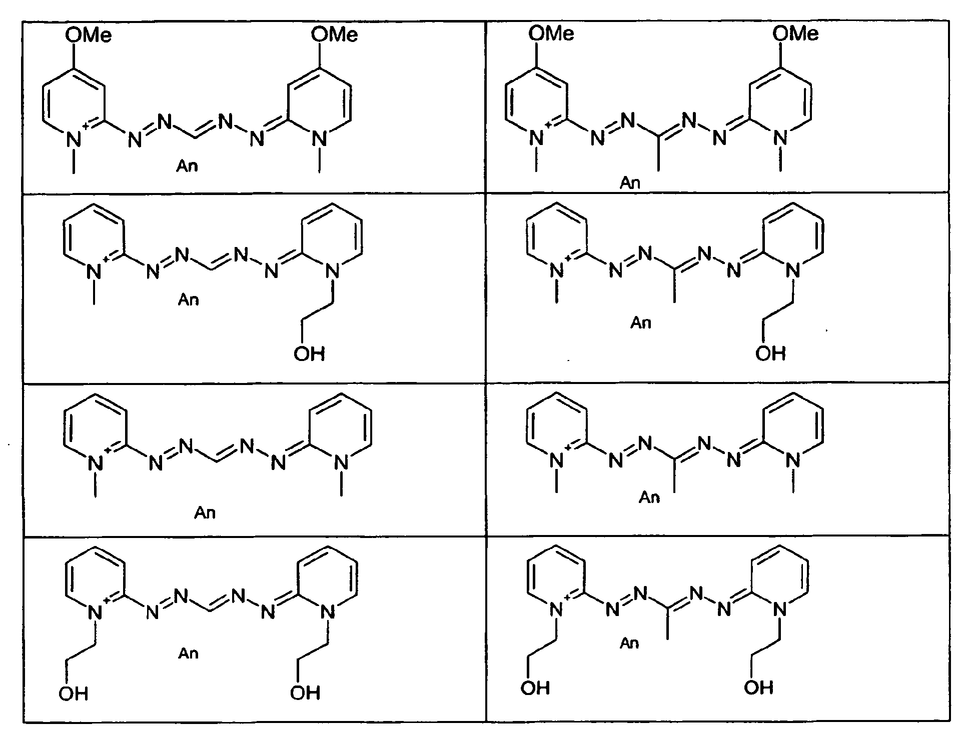

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition de la présente invention peut de plus contenir des bases d'oxydations et des coupleurs classiquement utilisés pour la coloration par oxydation.

A titre d'exemple, on peut citer les paraphénylènediamines, les bis-phénylalkylènediamines, les para-aminophénols, les ortho-aminophénols, les bases hétérocycliques et leurs sels d'addition avec un acide.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition avec un acide.

Lorsqu'ils sont présents, les bases et les coupleurs sont chacun généralement présents en quantité comprise entre 0,001 et 10 % en poids environ du poids total de la composition tinctoriale, de préférence entre 0,005 et 6 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Pour la teinture des fibres kératiniques humaines, le milieu de teinture est un milieu cosmétique approprié.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 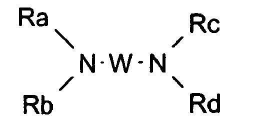 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe qui comprend l'application d'une composition tinctoriale contenant un colorant de formule (I) telle que définie précédemment sur les fibres kératiniques. Après un temps de pause, les fibres kératiniques sont rincées laissant apparaître des fibres colorées. Le temps de pause est généralement compris entre 3 à 50 minutes environ, de préférence 5 à 30 minutes environ.

Lorsque la composition tinctoriale comprend une base d'oxydation et/ou un coupleur, la composition tinctoriale peut alors contenir un agent oxydant. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

D'une façon générale, les composés de formule (I) sont préparés en mélangeant un composé de formule (RI') à (RVIII') et un composé de formule (VIII) en présence d'un oxydant, ces composés étant tels que définis ci-dessous.

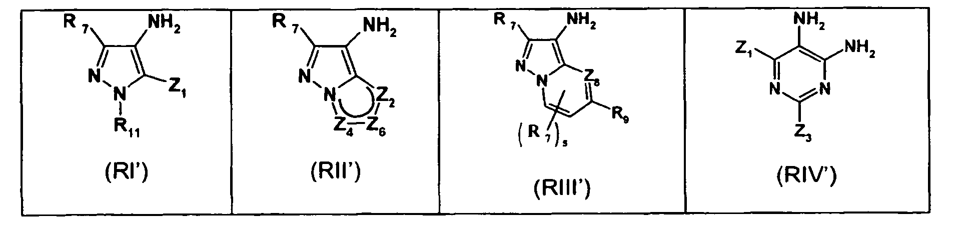

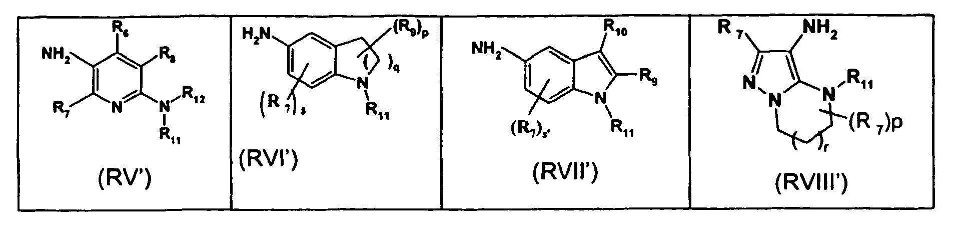

Dans ces formules, les radicaux sont tels que définis précédemment pour les formules RI, RII, RIII, RIV, RV, RVI, RVII et RVIII.

Le composé de formule (VIII) est représenté par 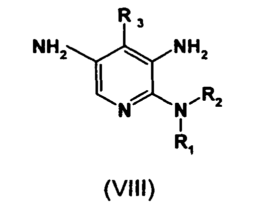 dans laquelle R₁, R₂ et R₃ ont les mêmes significations que précédemment.

Selon un mode de réalisation particulier, un composé de formule (RI') à (RVIII') est mélangé à un composé de formule (VIII) dans un solvant de point d'ébullition compris entre 60°C et 180°C, de préférence entre 60°C et 110°C, par exemple de l'acétonitrile, ou un alcool tel que le méthanol, l'éthanol ou un mélange de ceux-ci avec de l'eau. Au milieu réactionnel est ajouté, à une température comprise entre 0°C et 10°C, une base minérale ou organique, par exemple de la soude, de la potasse, une trialkylamine, en ajustant le pH entre 4 et 12, de préférence entre 7 et 9 ; puis, un oxydant organique ou minéral, par exemple de l'eau oxygénée, le nitrate de cérium(IV)ammonium (CAN), l'oxyde d'argent, ou un peracide tel que l'acide métaperbenzoïque, l'acide per-formique, l'acide per-acétique est ajouté au milieu. Une fois les réactifs consommés, de l'eau est ajoutée au milieu réactionnel et les phases organiques et aqueuses sont séparées par les méthodes classiques. La phase aqueuse est ensuite extraite plusieurs fois par du butanol, séchées sur du sulfate de sodium ou magnésium, puis distillée jusqu'à l'obtention d'un brut réactionnel sous forme de poudre ou d'huile. Ce résidu est purifié par recristallisation éventuellement fractionnée et/ou par chromatographie sur silice SiO₂.

Les composés de formule (RI') à (RVIII') sont des composés bien connus de la technique. En particulier, les composés de formule RI' sont obtenus selon les procédés de synthèse décrits dans les demandes de brevets FR 2 733 749, FR 2 827 601, DE 19 619 112. Les composés de formule Rll' peuvent être obtenus selon les procédés de synthèse décrits dans la demande de brevet EP 923 929. Les composés de formule Rlll' peuvent être obtenus selon les procédés de synthèse décrits dans les demandes de brevets EP 926 149 et FR 2 771 631. Les composés de formule RIV' peuvent être obtenus selon les procédés de synthèse décrits dans les demandes de brevet DE2613707, DE2554456 et DE2516117. Les composés de formule RV' peuvent être obtenus selon les procédés de synthèse décrits dans la demande de brevet FR 2 832 148. Les composés de formule RVI' peuvent être obtenus selon les procédés de synthèse décrits dans le la demande de brevet JP11158047. Les composés de formule RVII' peuvent être obtenus selon les procédés de synthèse décrits dans la demande de brevet EP 424 261. Les composés de formule RVIII' peuvent être obtenus selon les procédés de synthèse décrits dans les demandes de brevets EP 728 464 et EP 740 931, DE 4133957.

Les composés de formule VIII peuvent être obtenus selon les procédés de synthèse décrits dans la demande de brevet FR 1 397 551. On peut aussi synthétiser certains de ces composés selon le schéma de synthèse suivant : 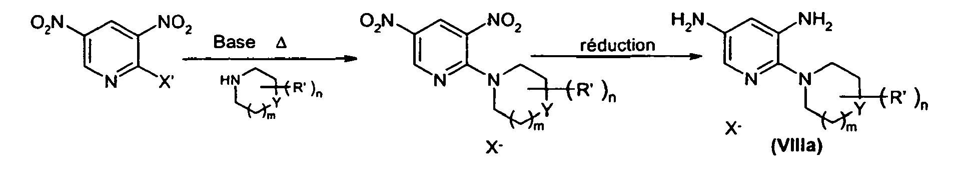 X' représentant un atome d'halogène tel qu'un chlore ou brome, ou un radical alcoxy en C1-C2 et R', Y, X-, n et m étant tels que définis précédemment.

Les composés nitrés peuvent être obtenus d'une façon générale en solubilisant sous agitation une 2-halogéno-3,5-dinitropyridine ou 2-alcoxy-3,5-dinitropyridine tel que la 2-chloro-3,5-dinitropyridine ou la 2-méthoxy-3,4-dinitropyridine dans un solvant protique ou aprotique de point d'ébullition compris entre 40°C et 180°C tel que par exemple le dichlorométhane, le dioxane, le DMF, le THF, un alcool inférieur, l'eau et en présence d'une base organique ou inorganique pouvant former un sel avec l'ion libéré. L'amine cyclique est ensuite introduite au goutte à goutte. La température du milieu réactionnel est en général comprise entre 25°C et 100°C. Après disparition des réactifs, le milieu réactionnel est refroidi à température ambiante et versé sur un mélange de glace et d'eau. Le précipité ainsi formé est essoré sur fritté, lavé à l'eau puis il est séché sous vide jusqu'à poids constant.

Les composés de formule (Vllla) peuvent ensuite être obtenus en réduisant les précurseurs nitrés soit par hydrogénation catalytique, soit par transfert d'hydrogène, soit par un métal tel que le zinc, l'étain ou le fer, soit par un hydrure tel que le borohydrure de sodium ou l'aluminohydrure de lithium. La réaction utilisée est de préférence l'hydrogénation catalytique hétérogène ou le transfert de phase par le cyclohexène. Le solvant est un solvant protique ou aprotique et de préférence le dichlorométhane ou un alcool de point d'ébullition compris entre 66°C et 160°C. Le catalyseur est classiquement le palladium sur charbon. La réaction d'hydrogénation est généralement réalisée à une température comprise entre 25°C et 80°C sous une pression d'hydrogène comprise entre 1 bar et 40 bars, de préférence entre 1 bar et 8 bars.

Les exemples qui suivent servent à illustrer l'invention sans toutefois présenter un caractère limitatif.

### EXEMPLES

### Exemple 1 : préparation du composé 3

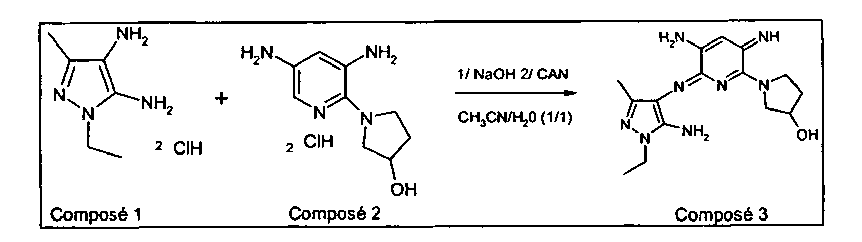

Dans un mélange de 10 ml d'acétonitrile et 10 ml d'eau, sous agitation et à température ambiante, on introduit 109 mg (0,51 mmoles ; 1 eq) de composé 1 et 100mg (0,374 mmoles ; 1 eq) de composé 2. De la soude 1M est alors coulée goutte à goutte afin de maintenir le pH entre 7 et 8, pendant que l'on ajoute 410mg de nitrate d'ammonium et de cérium (CAN, 0,748 mmoles ; 2 eq) dissous dans 10ml d'eau. La solution prend en quelques minutes une coloration noir-vert. On laisse réagir pendant 2 heures. La phase aqueuse est ensuite extraite au butanol : la phase organique est séchée sur sulfate de sodium puis évaporée sous vide. La solution est évaporée à sec puis le résidu obtenu est repris par de l'acétone (20ml) et de nouveau filtrée. On obtient ainsi 127,5 mg d'une poudre noire (composé 3) présentant les caractéristiques suivantes :
Masse (ESI +/-) : 331 (MH)+ , 329 (MH)-.
RMN 1 H (400 MHz, MeOD): 6,25 (s, 1 H);4,52 (s, 1 H);3,98 (m, 2H);3,87 (q, 2H);3,68 (m, 2H); 2,21 (s, 3H);2,07 (m, 2H); 1,31 (t, 3H).
Absorbance (acétonitrile): λₘₐₓ=408nm, λₘₐₓ=580nm

### Exemple 2 : préparation du composé 6

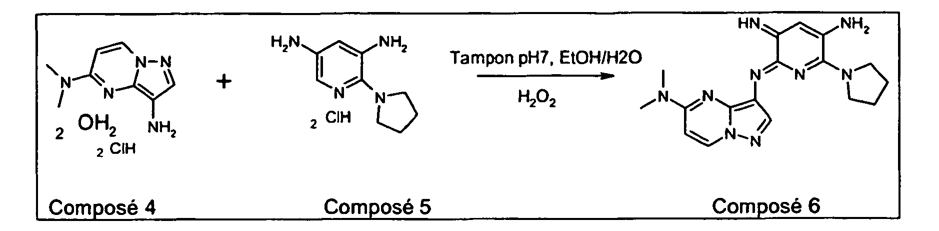

Dans 10 ml d'un mélange constitué d'éthanol, d'eau (30/70) et de tampon pH 7 (proportions respectives 4 :1), on dissout 99,9 mg (0,349 mmoles) de composé 4 et 99,6 mg (0,396 mmoles) de composé 5. Après solubilisation, 10 ml d'eau oxygénée à 20 volumes sont ajoutés. Le milieu réactionnel prend en 1 heure une teinte noire. Celui-ci est par la suite lyophilisé. Après purification par chromatographie sur phase inverse, évaporation des solvants d'élution et séchage, on récupère 85 mg d'un solide noir qui présente les caractéristiques suivantes :
Masse (ESI +/-) : 352 (MH)+
Absorbance (acétonitrile) : λ=447nm, λ=583nm.

### Exemple 3 : préparation du composé 8

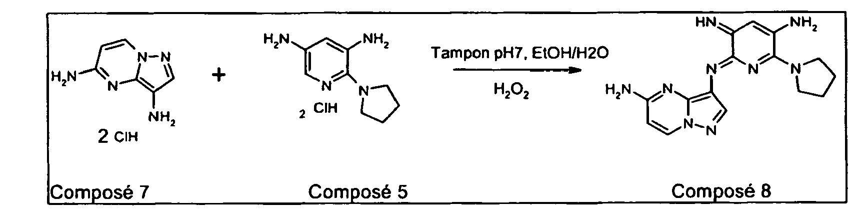

Dans 2 ml d'un mélange constitué d'éthanol, d'eau (30/70) et de tampon pH 7 (proportions respectives 4 :1), on dissout 18 mg (0,081 mmoles) de composé 7 et 20 mg (0,079 mmoles) de composé 8. Après solubilisation, 2 ml d'eau oxygénée à 20 volumes sont ajoutés. Le milieu réactionnel prend en 70 minutes une teinte noire. Celui-ci est par la suite lyophilisé. Après purification par chromatographie sur phase inverse, évaporation des solvants d'élution et séchage, on récupère 10 mg d'un solide noir qui présente les caractéristiques suivantes :
Masse (ESI +/-) : 324 (MH)+
RMN 1 H (400 MHz, CD3OD): 2.05 (m, 4H) ; 3.90 ppm (m, 4H) ; 5.96 (s, 1 H);6.40 (d, 1H);8,36 (d, 1H);8.74 (s, 1 H).
Absorbance (acétonitrile) : λ=442 nm, λ=539nom, λ=577nm

### Exemple 4 : préparation du composé 9

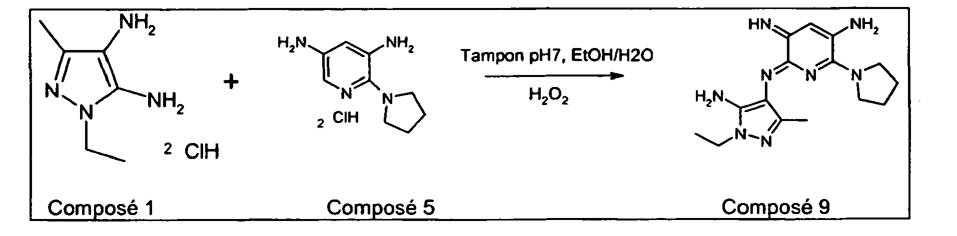

Dans 2 ml d'un mélange constitué d'éthanol, d'eau (30/70) et de tampon pH 7 (proportions respectives 4 :1), on dissout 16,6 mg (0,078 mmoles) de composé 1 et 20 mg (0,079 mmoles) de composé 5. Après solubilisation, 2 ml d'eau oxygénée à 20 volumes sont ajoutés. Le milieu réactionnel prend en 70 minutes une teinte noire. Celui-ci est par la suite lyophilisé. Après purification par chromatographie sur phase inverse, évaporation des solvants d'élution et séchage, on récupère 11 mg d'un solide noir qui présente les caractéristiques suivantes :
Masse (ESI +/-) : 315 (MH)+
RMN 1 H (400 MHz, CD3OD): 1.32 ppm (t, 3H) ; 1.99 ppm (m, 4H) ; 2.19 ppm (s, 3H) ; 3.79 (m, 4H).;3.87 (q, 2H);6 (s, 1 H).
Absorbance (acétonitrile) : λ=407nm, λ=581 nm.

### Exemple 5 : préparation du composé 11 :

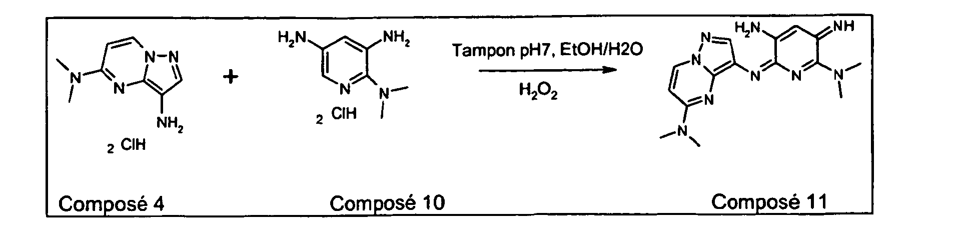

Dans 0,5 ml d'un mélange constitué d'éthanol, d'eau (30/70) et de tampon pH 7 (proportions respectives 4 :1), on dissout 5 mg (0,02 mmoles) de composé 4 et 4.5 mg (0,02 mmoles) de composé 10. Après solubilisation, 0,5 ml d'eau oxygénée à 20 volumes sont ajoutés. Le milieu réactionnel prend en 1 heure une teinte noire. Celui-ci est par la suite lyophilisé. Après purification par chromatographie sur phase inverse, évaporation des solvants d'élution et séchage, on récupère 3 mg d'un solide noir qui présente les caractéristiques suivantes :
Masse (ESI +/-) : 326 (MH)+
RMN 1 H (400 MHz, CD3OD): 3.29 ppm (s, 12H) ; 5.96 ppm (s, 1 H) ; 6.65 ppm (d, 1H) ; 8.39 ppm (d, 1H).;8.82 ppm (s, 1 H).
Absorbance (acétonitrile) : λ=457nm, λ=578nm

### Exemple 6 : préparation du composé 13

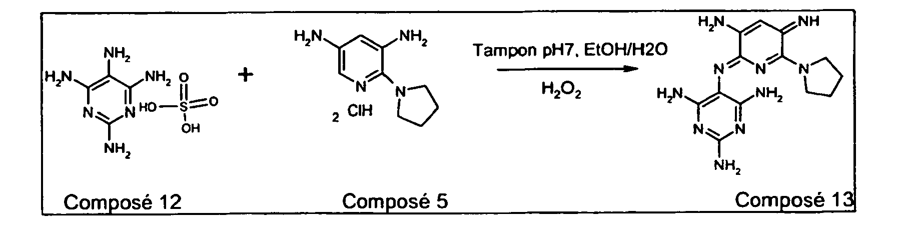

Dans 5 ml d'un mélange constitué d'éthanol, d'eau (30/70) et de tampon pH 7 (proportions respectives 4:1), on dissout 50 mg (0,21 mmoles) de composé 12 et 48 mg (0,019 mmoles) de composé 5. Après solubilisation, 5 ml d'eau oxygénée à 20 volumes sont ajoutés. Le milieu réactionnel prend en 70 minutes une teinte noire. Celui-ci est par la suite lyophilisé. Après purification par chromatographie sur phase inverse, évaporation des solvants d'élution et séchage, on récupère 11 mg d'un solide noir qui présente les caractéristiques suivantes :
Masse (ESI +/-) : 315 (MH)+
RMN 1 H (400 MHz, CD3OD): 2.01 ppm (m, 4H) ; 3.73 (m, 4H).;6.21 ppm (s, 1 H).
Absorbance (acétonitrile) : λ=435nm, λ=607nm

### Exemple 7: préparation du composé 15

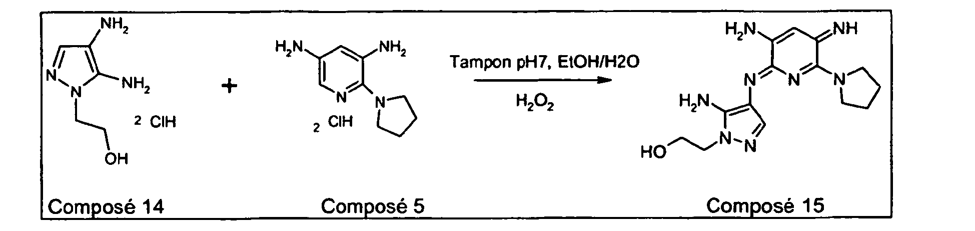

Dans 0,5 ml d'un mélange constitué d'éthanol, d'eau (30/70) et de tampon pH 7 (proportions respectives 4 :1), on dissout 4,3 mg (0,02 mmoles) de composé 14 et 5 mg (0,02 mmoles) de composé 5. Après solubilisation, on ajoute 0,5 ml d'eau oxygénée à 20 volumes. Le milieu réactionnel prend en 1 heures une teinte noire. Celui-ci est par la suite lyophilisé. Après purification par chromatographie sur phase inverse, évaporation des solvants d'élution et séchage, on récupère 3 mg d'un solide noir qui présente les caractéristiques suivantes :
Masse (ESI +/-) : 317 (MH)+
RMN 1H (400 MHz, CD3OD): 2.01 ppm (m, 4H) ; 3.86 ppm (m, 6H) ; 4.04 ppm (m, 2H) ; 5.91 ppm (s, 1H).;8.09 ppm (s, 1H).
Absorbance (acétonitrile) : λ=438nm, λ=571 nm

### Exemple 8 : préparation du composé 17

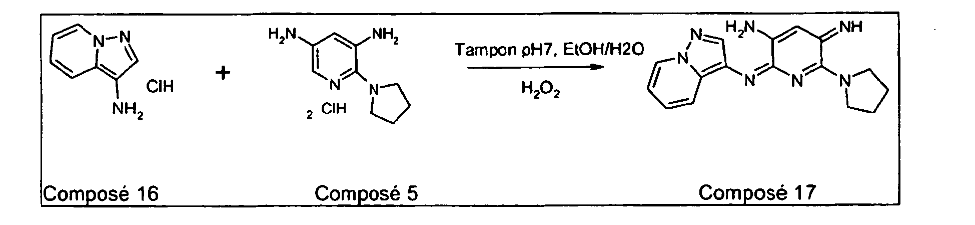

Dans 0,5 ml d'un mélange constitué d'éthanol, d'eau (30/70) et de tampon pH 7 (proportions respectives 4 :1), on dissout 3.3 mg (0,02 mmoles) de composé 16 et 5 mg (0,02 mmoles) de composé 5. Après solubilisation, on ajoute 0.5 ml d'eau oxygénée à 20 volumes. Le milieu réactionnel prend en 1 heure une teinte noire. Celui-ci est par la suite lyophilisé. Après purification par chromatographie sur phase inverse, évaporation des solvants d'élution et séchage, on récupère 3 mg d'un solide noir qui présente les caractéristiques suivantes :
Masse (ESI +/-) : 308 (MH)+
RMN 1 H (400 MHz, CD3OD): 2.07 ppm (m, 4H) ; 3.91 ppm (m, 4H) ; 6.06 ppm (s, 1 H) ; 7 ppm (m, 1H); 7.37 ppm (m, 1 H) ; 8.06 ppm (d, 1H); 8.49 ppm (d,1 H).;8.78 ppm (s, 1 H).
Absorbance (acétonitrile) : λ=448nm, λ=575nm

### Exemple 9 : préparation du composé 19

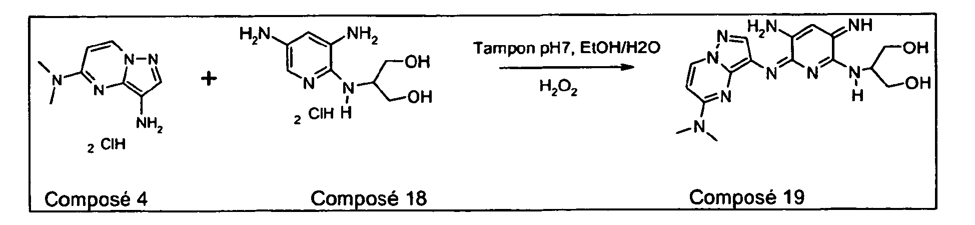

Dans 0,5 ml d'un mélange constitué d'éthanol, d'eau (30/70) et de tampon pH 7 (proportion respective 4 :1), on dissout 5 mg (0,02 mmoles) de composé 4 et 5.4 mg (0,02 mmoles) de composé 18. Après solubilisation, on ajoute 0,5 ml d'eau oxygénée à 20 volumes. Le milieu réactionnel prend en 1 heure une teinte noire. Celui-ci est par la suite lyophilisé. Après purification par chromatographie sur phase inverse, évaporation des solvants d'élution et séchage, on récupère 3 mg d'un solide noir qui présente les caractéristiques suivantes :
Masse (ESI +/-) : 372 (MH)+
RMN 1 H (400 MHz, CD3OD): 3.31 ppm (s, 6H) ; 3.93-3.82 ppm (m, 4H) ; 4.43 ppm (m, 1 H) ; 6.16 ppm (s, 1 H) ; 6.68 ppm (d, 1 H) ; 8.41 ppm (d, 1 H) ; 8.85 ppm (s, 1 H).
Absorbance (acétonitrile) : λ=451 nm, λ=575nm

### Exemple 10 : préparation du composé 21 :

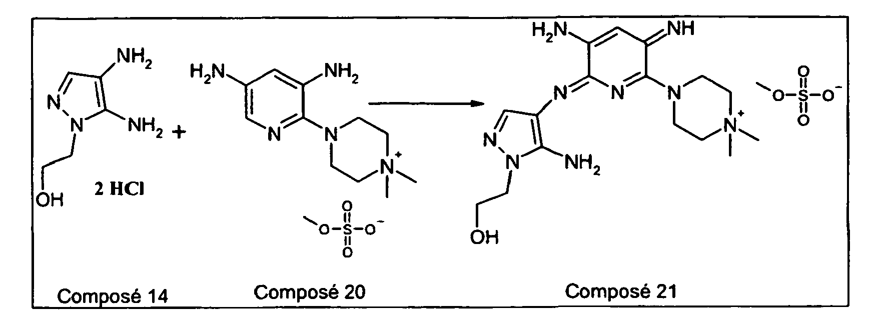

Le composé 20 (7,38 x 10⁻⁴ mol) est solubilisé dans l'eau (10ml). Le composé 14 (8,14 x 10-⁴ mol) est ajouté, puis le CAN (1,62 x 10⁻³ mol dans 1 ml d'eau). La soude (15% aq) est ajoutée pour obtenir un pH entre 7 et 8. Après 5 minutes, la solution devient noire. La solution est agitée pendant 24h à température ambiante et pH 7-8 (par addition d'aliquots de soude). La solution est évaporée à sec puis le résidu obtenu est repris par de l'acétone (20ml) et de nouveau évaporée. On obtient ainsi une poudre noire présentant les caractéristiques suivantes :
Masse (ESI +) : 361
CCM : Ethanol 2 : H₂O 1 : HOAc 1 Rf = 0.2 (noir) ;
Absorbance (H2O): λ=465nm, λ=568nm

### EXEMPLES DE TEINTURE

| **Exemples** | **1** | **2** | **3** | **4** | **5** |
|---|---|---|---|---|---|
| Composé 6 | 2.5.10⁻⁴ mole | | | | |
| Composé 8 | | 2.5.10⁻⁴ mole | | | |
| Composé 9 | | | 2.5.10⁻⁴ mole | | |
| Composé 11 | | | | 2.5.10⁻⁴ mole | |
| Composé 15 | | | | | 2.5.10⁻⁴ mole |
| Support de teinture (1) | (*) | (*) | (*) | (*) | (*) |
| Eau déminéralisée q.s.p. (g) | 100 | 100 | 100 | 100 | 100 |

| | | | | | |
|---|---|---|---|---|---|
| (*) Support de teinture (1) pH 7 Alcool éthylique à 96° 20,8 g Alkyl en C₈-C₁₀ polyglucoside en solution aqueuse à 60% 12 g Alcool benzylique 4,0 g Polyéthylène glycol à 8 motifs d'oxyde d'éthylène 6,0 g Na₂HPO₄ 0,28 g KH₂PO₄ 0,46 g | | | | | |

On obtient un pH final de 7.
Chaque mélange obtenu est appliqué sur des mèches de cheveux gris à 90 % de blancs. Après 30 min de pose, les mèches de cheveux sont rinçés, lavés avec un shampooing standard, rinçés à nouveau puis séchés.
Les résultats de teinture suivants ont été obtenus.

| **Exemples** | **1** | **2** | **3** | **4** | **5** |
|---|---|---|---|---|---|
| **Nuance observée** | Gris bleu intense | Brun intense | Gris vert intense | Gris violet intense | Gris bleu intense |

## Revendications

1. Composition tinctoriale comprenant, dans un milieu approprié, un composé de formule (I) suivante : 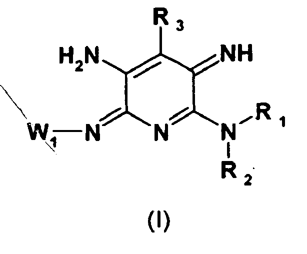 dans laquelle
• R₃ représente
- un atome d'hydrogène,
- une chaîne hydrocarbonée en C₁-C₁₀ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₃ ne comportant pas de liaison peroxyde, ni de radicaux diazo, nitro ou nitroso, R₃ n'étant pas relié au cycle pyridinique par un atome d'oxygène, d'azote ou de soufre ;
• R₁ et R₂ représentent indépendamment l'un de l'autre :
- un atome d'hydrogène
- une chaîne hydrocarbonée en C₁-C₁₀ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; R₁ et R₂ ne comportant pas de liaison peroxyde, ni de radicaux diazo, nitro ou nitroso, et R₁ et R₂ n'étant pas directement reliés à l'atome d'azote par un atome d'oxygène, de soufre ou d'azote.
- un radical cationique -D-Z dans lequel Z est un radical onium et D est une chaine hydrocarbonée en C₁-C₁₄ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂; le bras D n'étant pas relié à l'atome d'azote par un atome d'azote , d'oxygène ou de soufre ;
• R₁ et R₂ forment ensemble avec l'atome d'azote auquel ils sont rattachés un cycle de formule (IV): 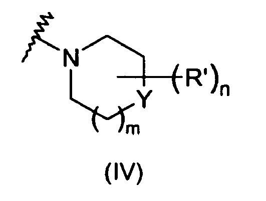 dans lequel :
• R' représente :
- un atome d'halogène ;
- un radical alkyle en C₁-C₄ éventuellement substitué par un ou plusieurs radicaux choisis parmi les radicaux hydroxy, carboxy, alcoxycarbonyle en C₁-C₄, alkyl(C₁-C₄)amido (alkyl(C₁-C₄)CONH-), alkyl(C₁-C₄)carboxamido, (alkyl(C₁-C₄) NHCO-), alkyl(C₁-C₄)sulfonyle (alkyl(C₁-C₄) SO₂-), alcoxy en C₁-C₄, alkyl(C₁-C₄)Sulfonamido ( alkyl(C₁-C₄)SO₂NH-), alkyl(C₁-C₄)sulfamoyle (alkyl(C₁-C₄) NH SO₂-);
- NR'₃R'₄ avec R'₃ et R'₄, identiques ou différents, représentent, un atome d'hydrogène ; un radical alkyle en C₁-C₄ éventuellement substitué par un ou plusieurs radicaux choisis parmi les radicaux hydroxy, alcoxy en C₁-C₄, amino, mono ou di alkylamino, alkyl(C₁-C₄)CO-, alkyl(C₁-C₄) NHCO-, alkyl(C₁-C₄)SO₂;
- un radical carboxy ;
- un radical alcoxycarbonyle en C₁-C₄ ;
- un radical alkyl(C₁-C₄)amido (alkyl(C₁-C₄)CONH-) ;
- un radical alkyl(C₁-C₄)SUIfonyle (alkylSO₂-)
- un radical alkylsulfonamido (alkyl(C₁-C₄) SO₂NH -) ;
- un radical hydroxy ;
- un radical alcoxy en C₁-C₄ ;
- un radical hydroxyalcoxy en C₂-C₄ ;
- un radical alkyl(C1-C4)carboxamido (alkyl(C1-C4) NHCO-) ;
- un radical alkyl(C₁-C₄)sulfamoyle (alkyl(C₁-C₄)-NH-SO₂-) ;
- un radical thioéther en C₁-C₄ ;
- un radical sulfonique (SO₃H) pouvant être sous forme de sel ;
- un radical cationique -D1-Z dans lequel D1 est une liaison covalente ou D,
- n est un nombre entier compris entre 0 et 12,
- m est un nombre entier compris entre 0 et 2,
- Y représente un atome de carbone, un atome d'oxygène, un radical NR'₅ ou un radical N⁺R'₆R'₇ avec R'₅ a la même signification que R'₃ ;R'₆ et R'₇ ont la même signification que R'₃ et sont différents d'un atome d'hydrogène,
étant entendu que lorsque n > 1, les radicaux R' peuvent être différents les uns des autres,
• W₁ représente un radical hétérocycle aromatique choisi parmi les radicaux suivants 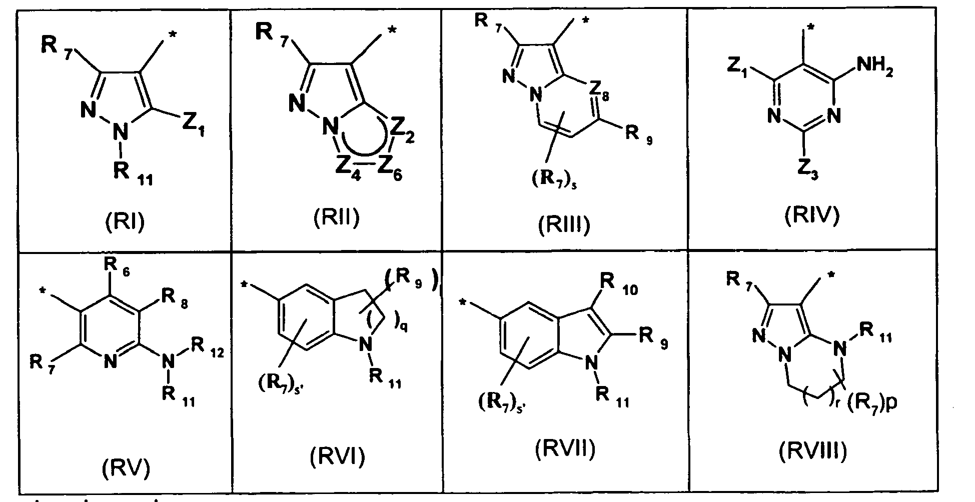 dans lesquels
• s est 0, 1 ou 2 ;
• s' est 0, 1, 2 ou 3
• Z₁ et Z₃, représentent indépendamment les uns des autres un radical hydroxy ou NR₁₁R₁₂
• Z₂, Z₄, Z₆ représentent indépendamment les uns des autres un atome d'azote, un radical CR₁₂ ou NR₁₁ sous réserve qu'au moins l'un d'entre eux représente un radical CR₁₂ et qu'il n'y est pas plus de 3 atomes d'azote contigus,
• Z₈ représente un atome d'azote, un radical CR₁₂,
• R₆ et R₈ indépendamment l'un de l'autre ont les mêmes significations que R₃.
• R₇, R₉, R₁₀, R₁₁ et R₁₂ représentent indépendamment les uns des autres
- un atome d'hydrogène,
- une chaîne hydrocarbonée en C₁-C₁₀ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène ; les radicaux R₇ et R₉ à R₁₂ ne comportant pas de liaison peroxyde, ni de radicaux diazo ou nitroso et les radicaux R₁₁ et R₁₂ n'étant pas reliés directement à l'atome d'azote par un atome d'oxygène, de soufre ou d'azote, étant entendu que les radicaux R₇ et R₉ peuvent être indépendants l'un de l'autre :
- p peut prendre les valeurs 4 à 8,
- q peut prendre les valeurs 1 à 3,
- r peut prendre les valeurs 0 à 2,
- * indique le point d'attache de W1 dans la formule (I),
étant entendu que le composé de formule (I) ne peut comporter plus d'un radical cationique D1-Z.

2. Composition selon la revendication 1 dans laquelle le composé (I) est tel que R₃ est choisis choisi parmi un atome d'hydrogène ou un radical alkyle en C₁-C₄ éventuellement substitué par un ou plusieurs radicaux choisis parmi les radicaux hydroxy, alcoxy en C₁-C₂, amino, (mono)- ou (di)alkylamino en C₁-C₂.

3. Composition selon la revendication 1 ou 2 dans laquelle le composé (I) est tel que R₃ est choisi parmi un atome d'hydrogène, un radical méthyle, éthyle, 2-hydroxyéthyle.

4. Composition selon la revendication 1, 2 ou 3 dans laquelle le composé (I) est tel que R₁ et R₂ sont choisis indépendamment choisis parmi l'hydrogène, un radical alkyle en C₁-C₆ éventuellement substitué par un hydroxy, un alkoxy, un amino, un (mono)- ou (di)alkylamino en C₁-C₄, un radical cationique -D-Z.

5. Composition selon l'une quelconque des revendications 1 à 4 dans laquelle le composé (1) est tel que R₁ ou R₂ sont indépendamment un radical -D-Z avec Z choisi parmi un imidazolium, un trialkyl(C₁-C₄)ammonium, un pyridinium, un pipéridinium.

6. Composition selon l'une quelconque des revendications 1 à 5 dans laquelle le composé (I) est tel que R₁ et R₂ sont choisis indépendamment parmi un radical hydrogène, méthyle, éthyle, hydroxyéthyle, propyle, propylimidazolium, propyltriméthylammonium.

7. Composition selon la revendication 1, 2 ou 3 dans laquelle le composé (I) est tel que R₁ et R₂, forment avec l'atome d'azote auquel ils sont rattachés un hétérocycle de 5 à 8 chaînons choisi parmi les hétérocycles pyrrolidine, pipéridine, homopipéridine, pipérazine, homopipérazine, diazépane.

8. Composition selon la revendication 7 dans laquelle le composé (I) est tel que R₁ et R₂ forment un hétérocycle choisi parmi la pyrrolidine, la 3-hydroxypyrrolidine, la 3-aminopyrrolidine, la 3-N,N-diméthylaminopyrrolidine, la 3-acétamidopyrrolidine, la 3-(méthylsulfonylamino)-pyrrolidine, la proline, la 3-hydroxyproline, le 3-(N-methylimidazolium)-pyrrolidine, le 3-(trialkyl(C1-C4)ammonium)pyrrolidine, la pipéridine, l'hydroxypipéridine, l'homopipéridine, le diazépane, N-méthyl homopipérazine, la N β-hydroxyéthylhomopipérazine et leurs sels d'addition.

9. Composition selon la revendication 7 dans laquelle le composé (1) est tel que R₁ et R₂ forment avec l'atome d'azote auquel ils sont rattachés un cycle pyrrolidinique éventuellement substitué.

10. Composition selon l'une quelconque des revendications 1 à 9 dans laquelle le composé de formule (I) est tel que le radical cationique -D-Z correspond à la formule (V) suivante : 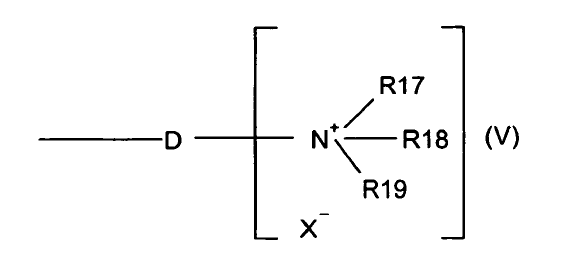 dans laquelle
• D est tel que défini à la revendication 1,
• R₁₇, R₁₈ et R₁₉, pris séparément, représentent un radical alkyle en C₁-C₁₅ un radical monohydroxyalkyle en C₁-C₆ un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, acylamino ou alkyl(C₁-C₆)sulfonyle ;
• R₁₇, R₁₈ et R₁₉ ensemble, deux à deux, peuvent conjointement former, avec l'atome d'azote quaternisé auxquel ils sont rattachés, un cycle saturé carboné à 5, 6 ou 7 chaînons pouvant contenir un ou plusieurs hétéroatomes, le cycle cationique pouvant être substitué par un atome d'halogène, un radical hydroxyle, un radical alkyle en C₁-C₆, un radical monohydroxyalkyle en C₁-C₆, un radical polyhydroxyalkyle en C₂-C₆, un radical alcoxy en C₁-C₆, un radical trial kyl(C₁-C₆)silanealkyle en C₁-C₆, un radical amido, un radical carboxyle, un radical alkyl(C₁-C₆)carbonyle, un radical thio, un radical thioalkyle en C₁-C₆, un radical alkyl(C₁-C₆)thio, un radical amino, un radical amino mono- ou di-substitué par un radical alkyl(C₁-C₆), alkyl(C₁-C₆)carbonyle, acylamino ou alkyl(C₁-C₆)sulfonyle;
• un des R17, R18 et R19 peut être relié à un des atomes de carbone ou d'azote du bras D pour former un cycle de 5 à 7 chainons, et
• X⁻ est un contre ion.

11. Composition selon la revendication 10 dans laquelle R₁₇, R₁₈ et R₁₉ séparément sont choisis parmi un radical alkyle en C₁-C₆, un radical monohydroxyalkyle en C₁-C₄, un radical polyhydroxyalkyle en C₂-C₄, un radical alcoxy(C₂-C₆)alkyle en C₁-C₄, un radical amidoalkyle en C₂-C₆, un radical trialkyl(C₁-C₆)silanealkyle en C₁-C₆ .

12. Composition selon la revendication 10 dans laquelle R₁₇ avec R₁₈ forment ensemble un cycle pyrrolidinium, pipéridinium, homopipéridinium, pipérazinium, homopipérazinium, morpholinium, R₁₉ est alors choisi parmi un radical alkyle en C₁-C₆; un radical monohydroxyalkyle en C₁-C₆ un radical polyhydroxyalkyle en C₂-C₆; un radical aminoalkyle en C₂-C₆, un radical aminoalkyle mono- ou di-substitué par un radical alkyl(C₁-C₆), alkyl(C₁-C₆)carbonyle; un radical carbamylalkyle en C₂-C₆ ; un radical trialkyl(C₁-C₆)silanealkyle en C₁-C₆ ; un radical alkyl(C₁-C₆)carboxyalkyle en C₁-C₆; un radical alkyl(C₁-C₆)carbonylalkyle en C₂-C₆ ; un radical N-alkyl(C₁-C₆)carbamytalkyle en C₂-C₆ .

13. Composition selon la revendication 10 dans laquelle R₁₇, R₁₈ et R₁₉ sont des radicaux alkyles.

14. Composition selon l'une quelconque des revendications 10 à 13 dans laquelle D est une chaîne alkylène en C₁-C₆ pouvant être substituée.

15. Composition selon l'une quelconque des revendications 1 à 9 dans laquelle le radical cationique -D-Z correspond à la formule (VI) 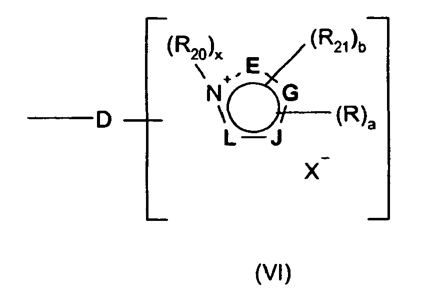 dans laquelle
• D est tel que défini à la revendication 1,
• les sommets E, G, J, L, identiques ou différents, représentent un atome de carbone, d'oxygène, de soufre ou d'azote pour former un cycle pyrazolium, imidazolium, triazolium, oxazolium, isooxazolium, thiazolium, isothiazolium,
• a est un nombre entier compris entre 1 et 3 inclus ;
• b est 0 ou 1 ;
• R, identiques ou différents, représentent un atome d'hydrogène, d'halogène, un radical alkyle en C₁-C₆, un radical monohydroxyalkyle en C₁-C₆, un radical polyhydroxyalkyle en C₂-C₆, un radical alcoxy en C₁-C₆, un radical amido, un radical carboxyle, un radical alkylcarbonyle en C₁-C₆, un radical thioalkyle en C₁-C₆, un radical alkyl(C₁-C₆)thio, un radical amino di substituée par un radical alkyl(C₁-C₆), alkyl(C₁-C₆)carbonyle, alkyl(C₁-C₆)sulfonyle, un radical benzyle, un radical phényle éventuellement substitué par un ou plusieurs radicaux choisis parmi les radicaux méthyle, hydroxy, amino, methoxy étant entendu que les radicaux R sont portés par un atome de carbone,
• R₂₁ représente un radical alkyle en C₁-C₆; un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₂-C₆, un radical alkyl(C₁-C₆)carboxyalkyle en C₁-C₆, un radical benzyle ; étant entendu que le radical R₂₁ est porté par un atome d'azote,
• R₂₀ représente un radical alkyle en C₁-C₆ ; un radical monohydroxyalkyle en C₁-C₆ ; un radical polyhydroxyalkyle en C₂-C₆ ; un radical benzyle ; un radical aminoalkyle en C₁-C₆ ; un radical aminoalkyle en C₁-C₆ dont l'amine est mono- ou di-subsitué par un radical alkyl(C₁-C₆), alkyl(C₁-C₆)carbonyle, acylamino ou alkyl(C₁-C₆)sulfonyle ; un radical carboxyalkyle en C₁-C₆ ; un radical carbamylalkyle en C₁-C₆; un radical trifluoroalkyle en C₂-C₆ ; un radical trialkyl(C₁-C₆)silanealkyle en C₁-C₆ ; un radical sulfonamidoalkyle en C₂-C₆ ; un radical alkyl(C₁-C₆)carboxyalkyle en C₁-C₆ un radical alkyl(C₁-C₆)sulfinylalkyle en C₂-C₆ ; un radical alkyl(C₁-C₆)sulfonylalkyle en C₂-C₆ ; un radical alkyl(C₁-C₆)carbonylalkyle en C₂-C₆ , un radical N-alkyl(C₁-C₆)carbamylalkyle en C₂-C₆ ; un radical N-alkyl(C₁-C₆)sulfonamidoalkyle en C₂-C₆ ;
• x est égal à 0 ou 1 ;
- lorsque x = 0, le bras de liaison D est rattaché à l'atome d'ammonium,
- lorsque x = 1, le bras de liaison D est rattaché à l'un des sommets E, G, J ou L;
• X⁻ est un contre-ion.

16. Composition selon la revendication 15 dans laquelle les sommets E, G, J et L forment un cycle imidazolium, pyrazolium, oxazolium, thiazolium et triazolium.

17. Composition selon la revendication 15 ou 16 dans laquelle la formule (VI) est telle que x est égal à 0 et D est une chaîne hydrocarbonée en C₁-C₄ pouvant être substituée.

18. Composition selon l'une quelconque des revendications 1 à 9 dans laquelle le radical cationique -D-Z correspond à la formule (VII) 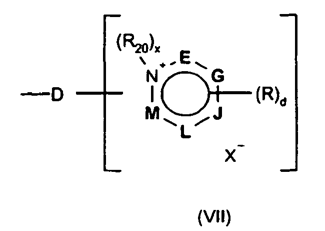 dans laquelle :
• D est tel que défini à la revendication 1,
• R, R₂₀ sont tels que définis à la revendication 17,
• les sommets E, G, J, L et M identiques ou différents, représentent un atome de carbone ou d'azote et forment un cycle choisi parmi les cycles pyridinium, pyrimidinium, pyrazinium et pyridazinium.
• d est un nombre entier compris entre 1 et 5 inclus ;
• x est égal à 0 ou 1
- lorsque x = 0, le bras de liaison D est rattaché à l'atome d'ammonium,
- lorsque x = 1, le bras de liaison D est rattaché à l'un des sommets E, G, J, L ou M,
• X- représente un contre ion.

19. Composition selon la revendication 18 dans laquelle dans la formule (VII), les sommets E,G,J,L et M avec l'azote quaternisé du cycle forment un cycle choisi parmi les cycles pyridinium, pyrimidinium, pyrazinium et pyridazinium.

20. Composition selon l'une quelconque des revendications 18 ou 19 dans laquelle dans la formule (VII), x est égal à 0 et R est choisi parmi un atome d'hydrogène, un radical alkyle en C₁-C₆, un radical monohydroxyalkyle en C₁-C₆, un radical polyhydroxyalkyle en C₂-C₆, un radical alcoxy en C₁-C₆, un radical alkylcarbonyle en C₁-C₆, un radical amino di-substitué par un radical alkyl(C₁-C₆), alkyl(C₁-C₆)carbonyte, alkyl(C₁-C₆)sulfonyle ; étant entendu que les radicaux R sont portés par un atome de carbone.

21. Composition selon l'une quelconque des revendications 18 ou 19 dans laquelle dans la formule (VII), x est égal à 1, R₂₀ est choisi parmi un radical alkyle en C₁-C₆ ; un radical monohydroxyalkyle en C₁-C₆ un radical polyhydroxyalkyle en C₂-C₆ ; un radical aminoalkyle en C₂-C₆, un radical aminoalkyle en C₂-C₆ dont l'amine est mono- ou di- substituée par un radical alkyl(C₁-C₆), alkyl(C₁-C₆)carbonyle, un radical acylamino ou un radical alkyl(C₁-C₆)sulfonyle ; un radical carbamylalkyle en C₁-C₆ un radical alkyl(C₁-C₆)carbonylalkyle en C₁-C₆ ; un radical N-alkyl(C₁-C₆)carbamylalkyle en C₁-C₆; R est choisi parmi un radical alkyle en C₁-C₆, un radical monohydroxyalkyle en C₁-C₆, un radical polyhydroxyalkyle en C₂-C₆, un radical alcoxy en C₁-C₆, un radical alkylcarbonyle en C₁-C₆, un radical amino di- substitué par un radical alkyl(C₁-C₆), alkyl(C₁-C₆)carbonyle, alkyl(C₁-C₆)sulfonyle.

22. Composition selon l'une quelconque des revendications 20 ou 21 dans laquelle dans la formule (VII), R₂₀ est un radical alkyle en C₁-C₄ pouvant être substitué par un radical hydroxy ou méthoxy et R un radical hydrogène, un radical alkyle en C₁-C₄ pouvant être substitué éventuellement par un radical hydroxy ou méthoxy.

23. Composition selon l'une quelconque des revendications 1 à 22 dans laquelle W₁ est choisi parmi les radicaux 5-aminopyrazole, 5-hydroxypyrazole, pyrazolo-[1,5-a]-pyridine, pyrazolo-[1,5-a]-pyrimidine, triaminopyrimidine et 2,3-diamino-6-alcoxy-pyridine.

24. Composition selon la revendication 23 dans laquelle W1 est choisi parmi les radicaux 5-aminopyrazole, 5-hydroxypyrazole.

25. Composition selon la revendication 24 dans laquelle W1 est choisi parmi les radicaux 5-aminopyrazole et 5-hydroxypyrazole avec R₇ et R₁₁ indépendamment choisis parmi un atome d'hydrogène ; une chaîne hydrocarbonée en C₁-C₄ linéaire ou ramifiée, pouvant former un ou cycles carbonés comportant de 5 à 6 chaînons, et pouvant être saturées ou insaturées, les atomes de carbone peuvent être, indépendamment les uns des autres, substitués par un ou plusieurs atomes d'halogène ou radicaux hydroxy, amino ; un radical alcoxy en C1-C2 ; un radical amino ; un radical (di)alkyl(C₁-C₄)amino.

26. Composition selon la revendication 23 dans laquelle W1 représente 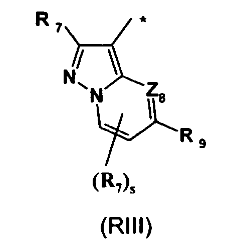 R₇, R₉ et Z₈ étant tels que définis aux revendications précédentes.

27. Composition selon la revendication 26 dans laquelle W1 est tel que Z₈ représente CR₁₂ alors R₇, R₉ et R₁₂ identiques ou différents sont de préférence choisis parmi :
• un atome d'hydrogène,
• un radical amino ,
• une chaîne hydrocarbonée en C₁-C₈ linéaire ou ramifiée, pouvant former un ou plusieurs cycles carbonés comportant de 5 à 8 chaînons, et pouvant être saturées ou insaturées, dont un ou plusieurs atomes de carbone de la chaîne carbonée peuvent être remplacés par un atome d'oxygène, d'azote ou de soufre ou par un groupement SO₂, et dont les atomes de carbone peuvent être, indépendamment les uns des autres, substitués par un ou plusieurs atomes d'halogène; les radicaux ne comportant pas de liaison peroxyde, ni de radicaux diazo ou nitroso et n'étant pas relié directement à l'atome d'azote par un atome d'oxygène, de soufre ou d'azote.

28. Composition selon la revendication 27 dans laquelle les radicaux R₇, R₉ et R₁₂ sont choisis parmi un atome d'hydrogène ; une chaîne hydrocarbonée en C₁-C₄ linéaire ou ramifiée et pouvant être saturées ou insaturées, les atomes de carbone peuvent être, indépendamment les uns des autres, substitués par un ou plusieurs atomes d'halogène ou radicaux hydroxy, amino ; un radical alcoxy en C1-C2; un radical amino ; un radical (di)alkyl(C₁-C₄)amino, éventuellement substitué.

29. Composition selon la revendication 26 dans laquelle W1 est tel que Z8 représente N et R₇ et R₉ sont choisis parmi un atome d'hydrogène ; un radical alkyle en C₁-C₆ linéaire ou ramifiée ; un radical monohydroxyalkyle en C₁-C₆ ; un radical polyhydroxyalkyle en C₂-C₆ ; un radical aminoalkyle en C₁-C₆, un radical aminoalkyle en C₁-C₆ dont l'amine est mono- ou di- substituée par un radical alkyl(C₁-C₆), alkyl(C₁-C₆)carbonyle ; un radical hydroxy ou amino, l'amino pouvant être éventuellement substitué par un ou plusieurs radicaux alkyles en C₁-C₁₀ linéaire ou ramifiée, pouvant former un ou plusieurs cycles carbonés comportant de 5 à 6 chaînons, et pouvant être saturées ou insaturées, les atomes de carbone peuvent être, indépendamment les uns des autres, substitués par un ou plusieurs atomes d'halogène ou radicaux hydroxy, amino ; un radical alcoxy en C1-C2.

30. Composition selon la revendication 26 dans laquelle R₇, R₉ et R₁₂ sont choisis parmi l'hydrogène, un radical alkyle en C₁-C₄ , un radical amino, un radical mono ou di alkylamino en C₁-C₄, un radical hydroxyalkyle en C₁-C₄; un radical alkoxy en C₁-C₄.

31. Composition selon l'une quelconque des revendications 1 à 30 dans laquelle le composé de formule (I) est un composé cationique substitué par un radical cationique D-Z.

32. Composition selon l'une quelconque des revendications 1 à 31 dans laquelle au moins un des radicaux R₁ et R₂ est un radical cationique -D-Z.

33. Composition selon l'une quelconque des revendications 1 à 31 dans laquelle R₁ et R₂ forment le cycle de formule (IV) dans lequel R' est un radical cationique D1-Z.

34. Composition selon la revendication 33 dans laquelle R' est choisi parmi un imidazolium ou un trialkylammonium.

35. Composition la revendication 1 dans laquelle Y est N⁺R'₆R'₇, R'₆ et R'₇ étant un radical alkyle en C₁-C₄.

36. Composition selon l'une quelconque des revendications précédentes dans laquelle le composé de formule (I) représente 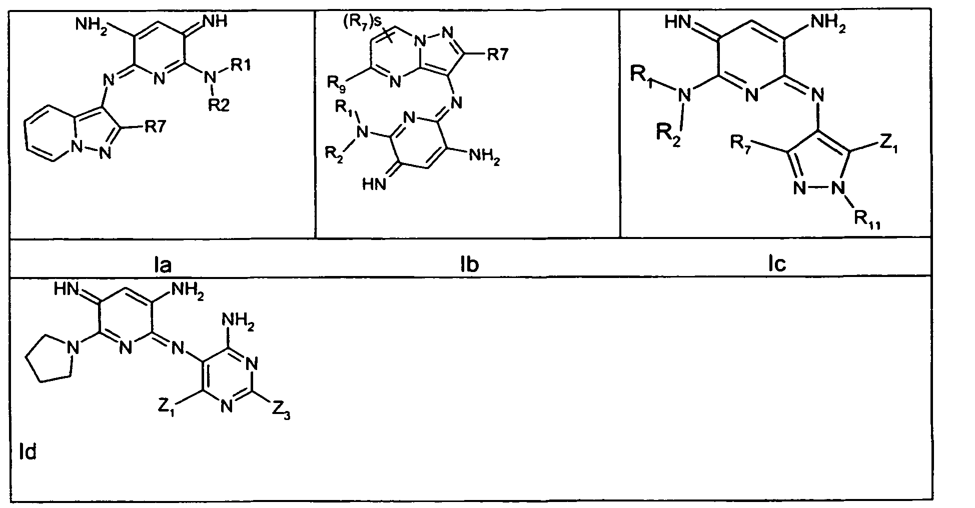 dans lesquelles R₁, R₂, R₆, R₇, R₈, R₉, R₁₁ Z₁, Z₂, Z₃ et s sont tels que définis aux revendications précédentes.

37. Composition selon l'une quelconque des revendications 1 à 36 dans laquelle le composé de formule (I) est choisi parmi

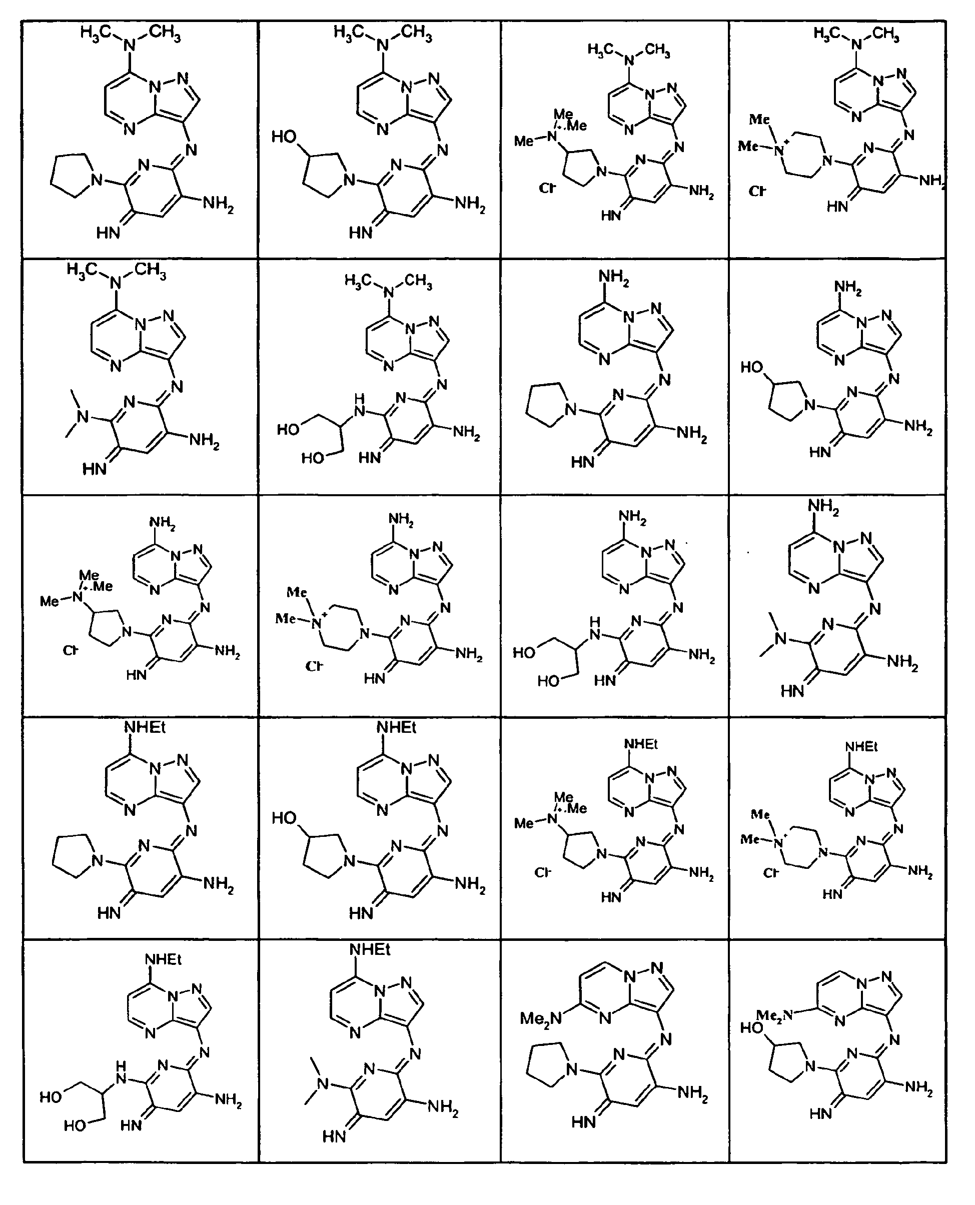

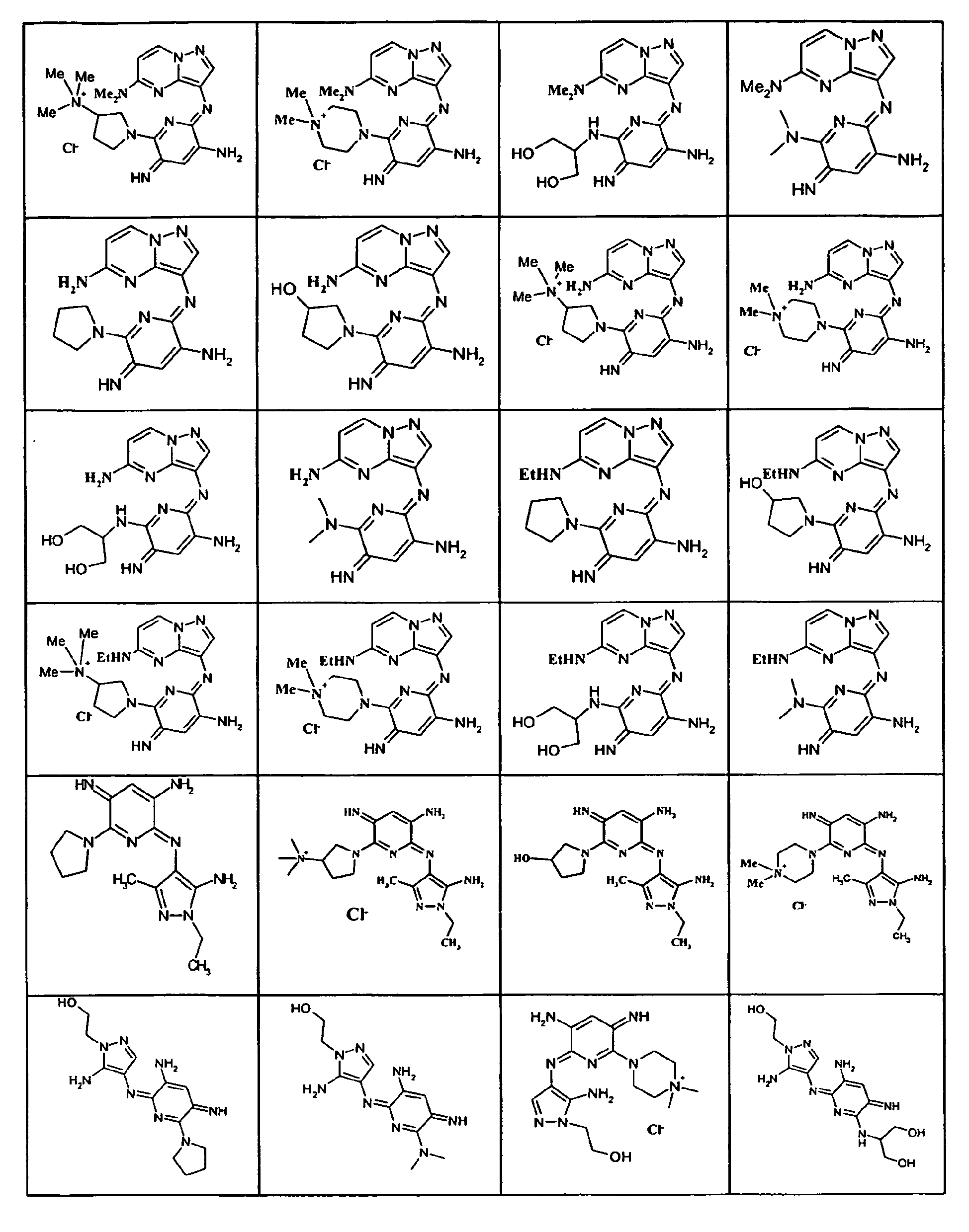

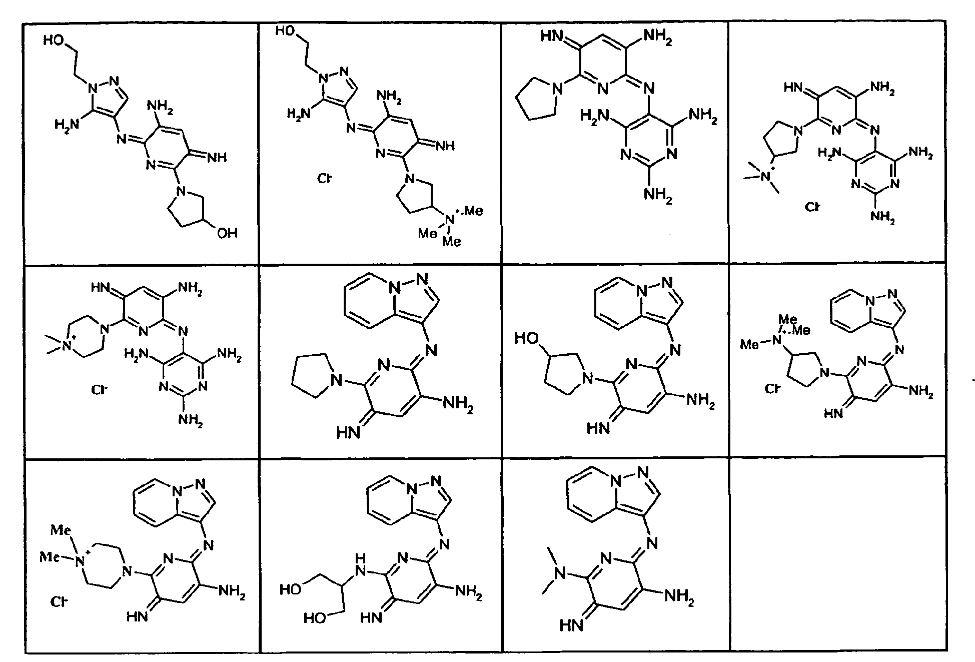

38. Composition selon l'une quelconque des revendications 1 à 37 dans laquelle la quantité de colorant (1) est comprise entre 0,01 et 10 % en poids.

39. Composition selon l'une quelconque des revendications 1 à 38 comprenant de plus une base d'oxydation choisie parmi les paraphénylènediamines, les bis-phénylalkylènediamines, les para-aminophénols, les ortho-aminophénols, les bases hétérocycliques, et leurs sels d'addition avec un acide.

40. Composition selon la revendication 39 dans laquelle la ou les bases d'oxydation sont présentes en quantité comprise entre 0,001 et 10 %.

41. Composition selon l'une quelconque des revendications 1 à 40 comprenant au moins un coupleur choisi parmi métaphénylènediamines, les méta-aminophénols, les métadiphénols, les coupleurs naphtaléniques et les coupleurs hétérocycliques et leur sel d'addition avec un acide.

42. Composition selon l'une quelconque des revendications 1 à 41 comprenant de plus un agent oxydant.

43. Colorant direct de formule (I) tel que défini dans l'une quelconque des revendications 1 à 37.

44. Procédé de coloration des fibres kératiniques qui comprend l'application de la composition selon l'une quelconque des revendications 1 à 42 pendant un temps suffisant pour obtenir la coloration désirée.

## Claims

1. Dye composition comprising, in a suitable medium, a compound of formula (I) below: 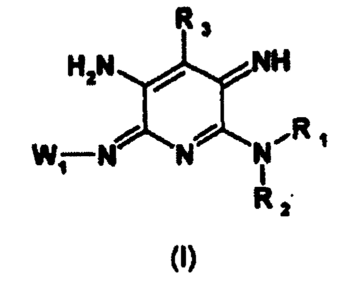 in which
• R₃ represents:
- a hydrogen atom,
- a linear or branched C₁-C₁₀ hydrocarbon-based chain, which can form one or more 5- to 8-membered carbon-based rings, and which may be saturated or unsaturated, one or more carbon atoms of the carbon-based chain of which may be replaced with an oxygen, nitrogen or sulfur atom or with an SO₂ group, and the carbon atoms of which may be, independently of each other, substituted with one or more halogen atoms; R₃ not comprising a peroxide bond or diazo, nitro or nitroso radicals, R₃ not being linked to the pyridine ring via an oxygen, nitrogen or sulfur atom;
• R₁ and R₂ represent, independently of each other:
- a hydrogen atom,
- a linear or branched C₁-C₁₀ hydrocarbon-based chain, which can form one or more 5- to 8-membered carbon-based rings, and which may be saturated or unsaturated, one or more carbon atoms of the carbon-based chain of which may be replaced with an oxygen, nitrogen or sulfur atom or with an SO₂ group, and the carbon atoms of which may be, independently of each other, substituted with one or more halogen atoms; R₁ and R₂ not comprising a peroxide bond or diazo, nitro or nitroso radicals, and R₁ and R₂ not being directly linked to the nitrogen atom via an oxygen, sulfur or nitrogen atom
- a cationic radical -D-Z in which Z is an onium radical and D is a linear or branched C₁-C₁₄ hydrocarbon-based chain, which can form one or more 5- to 8-membered carbon-based rings, and which may be saturated or unsaturated, one or more carbon atoms of the carbon-based chain of which may be replaced with an oxygen, nitrogen or sulfur atom or with an SO₂ group; the arm D not being linked to the nitrogen atom via a nitrogen, oxygen or sulfur atom;
• R₁ and R₂ form, together with the nitrogen atom to which they are attached, a ring of formula (IV): 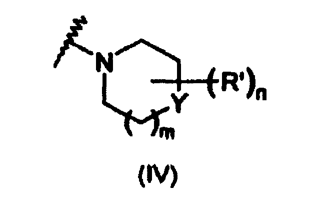 in which
• R' represents:
- a halogen atom;
- a C₁-C₄ alkyl radical optionally substituted with one or more radicals chosen from hydroxyl, carboxyl, C₁-C₄ alkoxycarbonyl, (C₁-C₉) alkylamido ((C₁-C₄) alkylCONH-), (C₁-C₄) alkylcarboxamido, ((C₁-C₄) alkylNHCO-), (C₁-C₄) alkylsulfonyl ((C₁-C₄) alkylSO₂-), C₁-C₄ alkoxy, (C₁-C₄) alkylsulfonamido ((C₁-C₄) alkylSO₂NH-) and (C₁-C₄) alkylsulfamoyl ((C₁-C₄) alkylNHSO₂-) radicals;
- NR'₃R'₄ with R'₃ and R'₄, which may be identical or different, representing a hydrogen atom, a C₁-C₄ alkyl radical optionally substituted with one or more radicals chosen from hydroxyl, C₁-C₄ alkoxy, amino, mono- or dialkylamino, (C₁-C₄)alkylCO-, (C₁-C₄) alkylNHCO- and (C₁-C₄)alkylSO₂ radicals;
- a carboxyl radical;
- a C₁-C₄ alkoxycarbonyl radical;
- a (C₁-C₄) alkylamido radical ((C₁-C₄) alkylCONH-) ;
- a (C₁-C₄) alkylsulfonyl radical (alkylSO₂-);
- an alkylsulfonamido radical ((C₁-C₄) alkylSO₂NH-) ;
- a hydroxyl radical;
- a C₁-C₄ alkoxy radical;
- a C₂-C₄ hydroxyalkoxy radical;
- a (C₁-C₄)alkylcarboxamido radical ((C₁-C₄) alkylNHCO-);
- a (C₁-C₄) alkylsulfamoyl ((C₁-C₄) alkyl-NH-SO₂-) radical;
- a C₁-C₄ thioether radical;
- a sulfonic radical (SO₃H), which may be in salt form;
- a cationic radical -D1-Z in which D1 is a covalent bond or D,
- n is an integer between 0 and 12,
- m is an integer between 0 and 2,
- Y represents a carbon atom, an oxygen atom, a radical NR'₅ or a radical N+R'₆R'₇ withR'₅ having the same meaning as R'₃; R'₆ and R'₇ have the same meaning as R'₃ and are other than a hydrogen atom,
it being understood that when n > 1, the radicals R' may be different from each other,
• W₁ represents an aromatic heterocyclic radical chosen from the following radicals 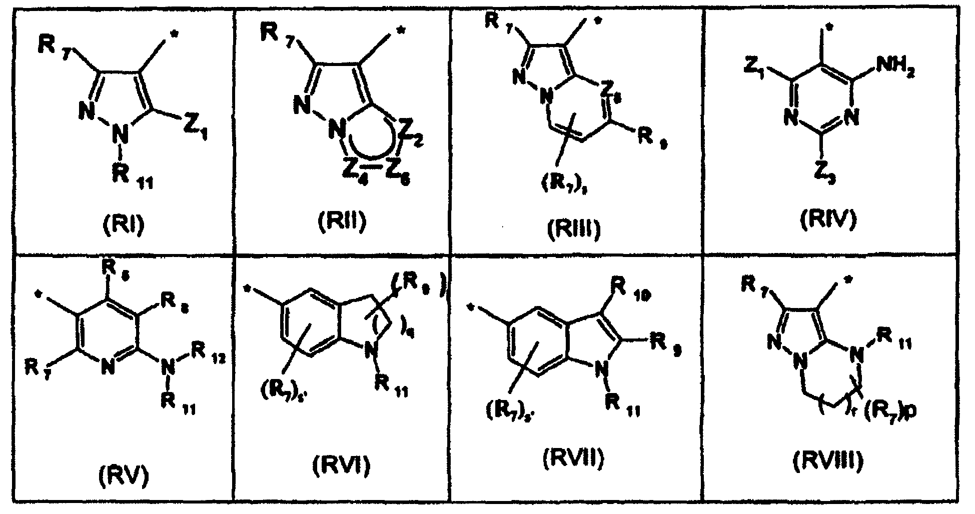 in which
• s is 0, 1 or 2,
• s' is 0, 1, 2 or 3,
• Z₁ and Z₃ represent, independently of each other, a hydroxyl or NR₁₁R₁₂ radical,
• Z₂, Z₄ and Z₆ represent, independently of each other, a nitrogen atom or a radical CR₁₂ or NR₁₁, with the proviso that at least one of them represents a radical CR₁₂ and that there are not more than three contiguous nitrogen atoms,
• Z₈ represents a nitrogen atom or a radical CR₁₂,
• R₆ and R₈, independently of each other, have the same meanings as R₃,
• R₇, R₉, R₁₀, R₁₁ and R₁₂ represent, independently of each other:
- a hydrogen atom,
- a linear or branched C₁-C₁₀ hydrocarbon-based chain, which can form one or more 5- to 8-membered carbon-based rings, and which may be saturated or unsaturated, one or more carbon atoms of the carbon-based chain of which may be replaced with an oxygen, nitrogen or sulfur atom or with an SO₂ group, and the carbon atoms of which may be, independently of each other, substituted with one or more halogen atoms; the radicals R₇ and R₉ to R₁₂ not comprising a peroxide bond or diazo or nitroso radicals, and the radicals R₁₁ and R₁₂ not being directly linked to the nitrogen atom via an oxygen, sulfur or nitrogen atom,
it being understood that the radicals R₇ and R₉ may be independent of each other,
- p may take the values 4 to 8,
- q may take the values 1 to 3, and
- r may take the values 0 to 2,
- * indicates the point of attachment of W₁ in formula (I),
it being understood that the compound of formula (I) cannot comprise more than one cationic radical D1-Z.

2. Composition according to Claim 1, in which compound (I) is such that R₃ is chosen from a hydrogen atom and a C₁-C₄ alkyl radical optionally substituted with one or more radicals chosen from hydroxyl, C₁-C₂ alkoxy, amino and (mono)- or (di)(C₁-C₂)alkylamino radicals.

3. Composition according to Claim 1 or 2, in which compound (I) is such that R₃ is chosen from a hydrogen atom and a methyl, ethyl or 2-hydroxyethyl radical.

4. Composition according to Claim 1, 2 or 3, in which compound (1) is such that R₁ and R₂ are independently chosen from hydrogen, a C₁-C₆ alkyl radical optionally substituted with a hydroxyl, an alkoxy, an amino, a (mono)- or (di)(C₁-C₄)alkylamino radical and a cationic radical -D-Z.

5. Composition according to any one of Claims 1 to 4, in which compound (I) is such that R₁ and R₂ are independently a radical -D-Z with Z chosen from an imidazolium, a tri(C₁-C₄)alkylammonium, a pyridinium and a piperidinium.

6. Composition according to any one of Claims 1 to 5, in which compound (I) is such that that R₁ and R₂ are independently chosen from hydrogen, methyl, ethyl, hydroxyethyl, propyl, propylimidazolium and propyltrimethylammonium radicals.

7. Composition according to Claim 1, 2 or 3, in which compound (I) is such that R₁ and R₂ form, with the nitrogen atom to which they are attached, a 5- to 8-membered heterocycle chosen from pyrrolidine, piperidine, homopiperidine, piperazine, homopiperazine and diazepane heterocycles.

8. Composition according to Claim 7, in which compound (I) is such that R₁ and R₂ form a heterocycle chosen from pyrrolidine, 3-hydroxypyrrolidine, 3-aminopyrrolidine, 3-N,N-dimethylaminopyrrolidine, 3-acetamidopyrrolidine, 3-(methylsulfonyl-amino)pyrrolidine, proline, 3-hydroxyproline, 3-(N-methylimidazolium)pyrrolidine, 3-(trialkyl(C₁-C₄)-ammonium)pyrrolidine, piperidine, hydroxypiperidine, homopiperidine, diazepane, N-methylhomopiperazine and N-β-hydroxyethylhomopiperazine, and the addition salts thereof.

9. Composition according to Claim 7, in which compound (I) is such that R₁ and R₂ form, with the nitrogen atom to which they are attached, an optionally substituted pyrrolidine ring.

10. Composition according to any one of Claims 1 to 9, in which the compound of formula (I) is such that the cationic radical -D-Z corresponds to formula (V) below: 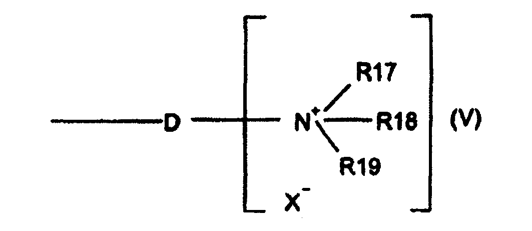 in which
• D is as defined in Claim 1,
• R₁₇, R₁₈ and R₁₉, taken separately, represent a C₁-C₁₅ alkyl radical; a C₁-C₆ monohydroxyalkyl radical; a C₂-C₆ polyhydroxyalkyl radical; a (C₁-C₆) alkoxy (C₁-C₆) alkyl radical; an aryl radical; a benzyl radical; a C₁-C₆ amidoalkyl radical; a tri (C₁-C₆) alkylsilane (C₁-C₆) alkyl radical; a C₁-C₆ aminoalkyl radical; a C₁-C₆ aminoalkyl radical in which the amine is mono- or disubstituted with a C₁-C₄ alkyl, (C₁-C₆)alkylcarbonyl, acylamino or (C₁-C₆)alkylsulfonyl radical;
• R₁₇, R₁₈ and R₁₉ together, in pairs, may form, with the quaternized nitrogen atom to which they are attached, a 5-, 6- or 7-membered carbon-based saturated ring which may contain one or more hetero atoms, the cationic ring possibly being substituted with a halogen atom, a hydroxyl radical, a C₁-C₆ alkyl radical, a C₁-C₆ monohydroxyalkyl radical, a C₂-C₆ polyhydroxyalkyl radical, a C₁-C₆ alkoxy radical, a tri (C₁-C₆) alkylsilane (C₁-C₆) alkyl radical, an amido radical, a carboxyl radical, a C₁-C₆ alkylcarbonyl radical, a thio radical, a C₁-C₆ thioalkyl radical, a (C₁-C₆)alkylthio radical, an amino radical or an amino radical mono- or disubstituted with a (C₁-C₆) alkyl, (C₁-C₆) alkylcarbonyl, acylamino or (C₁-C₆)alkylsulfonyl radical;
• one from among R₁₇, R₁₈ and R₁₉ may be linked to one of the carbon or nitrogen atoms of the arm D to form a 5- to 7-membered ring, and
• X⁻ is a counterion.

11. Composition according to Claim 10, in which R₁₇, R₁₈ and R₁₉, separately, are chosen from a C₁-C₆ alkyl radical, a C₁-C₄ monohydroxyalkyl radical, a C₂-C₄ polyhydroxyalkyl radical, a (C₂-C₆)alkoxy(C₁-C₄)alkyl radical, a C₂-C₆ amidoalkyl radical and a tri(C₁-C₆)alkylsilane(C₁-C₆)alkyl radical.

12. Composition according to Claim 10, in which R₁₇ and R₁₈ together form a pyrrolidinium, piperidinium, homopiperidinium, piperazinium, homopiperazinium or morpholinium ring, and R₁₉ is then chosen from a C₁-C₆ alkyl radical; a C₁-C₆ monohydroxyalkyl radical; a C₂-C₆ polyhydroxyalkyl radical; a C₂-C₆ aminoalkyl radical; an aminoalkyl radical mono- or disubstituted with a (C₁-C₆) alkyl or (C₁-C₆) alkylcarbonyl radical; a C₂-C₆ carbamylalkyl radical; a tri (C₁-C₆) alkylsilane (C₁-C₆) alkyl radical; a (C₁-C₆) alkylcarboxy (C₁-C₆) alkyl radical; a (C₁-C₆) alkylcarbonyl (C₂-C₆) alkyl radical; an N- (C₁-C₆) alkylcarbamyl (C₂-C₆) alkyl radical.

13. Composition according to Claim 10, in which R₁₇, R₁₈ and R₁₉ are alkyl radicals.

14. Composition according to any one of Claims 10 to 13, in which D is a C₁-C₆ alkylene chain that may be substituted.

15. Composition according to any one of Claims 1 to 9, in which the cationic radical -D-Z corresponds to formula (VI) 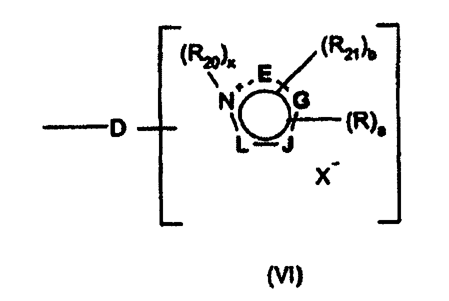 in which
• D is as defined in Claim 1,
• the ring members E, G, J and L, which may be identical or different, represent a carbon, oxygen, sulfur or nitrogen atom to form a pyrazolium, imidazolium, triazolium, oxazolium, isoxazolium, thiazolium or isothiazolium ring,
• a is an integer between 1 and 3 inclusive;
• b is 0 or 1;
• R, which may be identical or different, represent a hydrogen or halogen atom, a C₁-C₆ alkyl radical, a C₁-C₆ monohydroxyalkyl radical, a C₂-C₆ polyhydroxyalkyl radical, a C₁-C₆ alkoxy radical, an amido radical, a carboxyl radical, a C₁-C₆ alkylcarbonyl radical, a C₁-C₆ thioalkyl radical, a (C₁-C₆)alkylthio radical, an amino radical disubstituted with a (C₁-C₆) alkyl, (C₁-C₆)alkylcarbonyl or (C₁-C₆)alkylsulfonyl radical, a benzyl radical, a phenyl radical optionally substituted with one or more radicals chosen from methyl, hydroxyl, amino and methoxy radicals; it being understood that the radicals R are borne by a carbon atom,
• R₂₁ represents a C₁-C₆ alkyl radical, a Cₗ-C₆ monohydroxyalkyl radical, a C₂-C₆ polyhydroxyalkyl radical, a tri(C₁-C₆)alkylsilane (C₁-C₆) alkyl radical, a (C₁-C₆) alkoxy (C₁-C₆)alkyl radical, a C₂-C₆ carbamylalkyl radical, a (C₁-C₆) alkylcarboxy (C₁-C₆) alkyl radical or a benzyl radical; it being understood that the radical R₂₁ is borne by a nitrogen atom,
• R₂₀ represents a C₁-C₆ alkyl radical; a C₁-C₆ monohydroxyalkyl radical; a C₂-C₆ polyhydroxyalkyl radical; a benzyl radical; a (C₁-C₆)aminoalkyl radical; a (C₁-C₆)aminoalkyl radical in which the amine is mono- or disubstituted with a (C₁-C₆)alkyl, (C₁-C₆)alkylcarbonyl, acylamino or (C₁-C₆)alkylsulfonyl radical; a carboxy(C₁-C₆)alkyl radical; a carbamyl(C₁-C₆)alkyl radical; a (C₂-C₆)trifluoroalkyl radical; a tri(C₁-C₆)alkylsilane(C₁-C₆)alkyl radical; a sulfonamido (C₂-C₆) alkyl radical; a (C₁-C₆) alkylcarboxy (C₁-C₆) alkyl radical; a (C₁-C₆)alkylsulfinyl(C₂-C₆)alkyl radical; a (C₁-C₆)alkylsulfonyl(C₂-C₆)alkyl radical; a (C₁-C₆) alkylcarbonyl (C₂-C₆) alkyl radical; an N-(C₁-C₆) alkylcarbamyl (C₂-C₆) alkyl radical; an N-(C₁-C₆) alkylsulfonamido (C₂-C₆) alkyl radical;
• x is equal to 0 or 1,
- when x = 0, the linker arm D is attached to the ammonium atom,
- when x = 1, the linker arm D is attached to one of the ring members E, G, J or L,
• X⁻ is a counterion.

16. Composition according to Claim 15, in which the ring members E, G, J and L form an imidazolium, pyrazolium, oxazolium, thiazolium or triazolium ring.

17. Composition according to Claim 15 or 16, in which formula (VI) is such that x is equal to 0 and D is a C₁-C₄ hydrocarbon-based chain that may be substituted.

18. Composition according to any one of Claims 1 to 9, in which the cationic radical -D-Z corresponds to formula (VII) 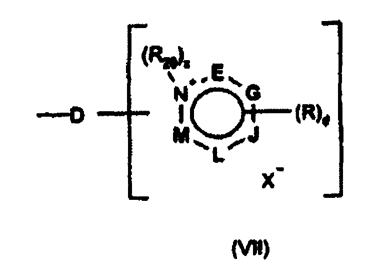 in which
• D is as defined in Claim 1,
• R and R₂₀ are as defined in Claim 17,
• the ring members E, G, J, L and M, which may be identical or different, represent a carbon or nitrogen atom and form a ring chosen from pyridinium, pyrimidinium, pyrazinium and pyridazinium rings,
• d is an integer between 1 and 5 inclusive,
• x is equal to 0 or 1,
- when x = 0, the linker arm D is attached to the ammonium atom,
- when x = 1, the linker arm D is attached to one of the ring members E, G, J, L or M,
• X⁻ represents a counterion.

19. Composition according to Claim 18, in which, in formula (VII), the ring members E, G, J, L and M form, with the quaternized nitrogen of the ring, a ring chosen from pyridinium, pyrimidinium, pyrazinium and pyridazinium rings.

20. Composition according to either of Claims 18 and 19, in which, in formula (VII), x is equal to 0 and R is chosen from a hydrogen atom, a C₁-C₆ alkyl radical, a C₁-C₆ monohydroxyalkyl radical, a C₂-C₆ polyhydroxyalkyl radical, a C₁-C₆ alkoxy radical, a C₁-C₆ alkylcarbonyl radical, an amino radical disubstituted with a (C₁-C₆) alkyl, (C₁-C₆) alkylcarbonyl or (C₁-C₆) alkylsulfonyl radical; it being understood that the radicals R are borne by a carbon atom.

21. Composition according to either of Claims 18 and 19, in which, in formula (VII), x is equal to 1, R₂₀ is chosen from a C₁-C₆ alkyl radical; a C₁-C₆ monohydroxyalkyl radical; a C₂-C₆ polyhydroxyalkyl radical; a C₂-C₆ aminoalkyl radical, a C₂-C₆ aminoalkyl radical in which the amine is mono- or disubstituted with a (C₁-C₆) alkyl, (C₁-C₆) alkylcarbonyl, acylamino or (C₁-C₆)alkylsulfonyl radical; a C₁-C₆ carbamylalkyl radical; a (C₁-C₆) alkylcarbonyl (C₁-C₆) alkyl radical; an N-(C₁-C₆)alkylcarbamyl(C₁-C₆)alkyl radical; R is chosen from a C₁-C₆ alkyl radical, a C₁-C₆ monohydroxyalkyl radical, a C₂-C₆ polyhydroxyalkyl radical, a C₁-C₆ alkoxy radical, a C₁-C₆ alkylcarbonyl radical, an amino radical disubstituted with a (C₁-C₆) alkyl, (C₁-C₆) alkylcarbonyl or (C₁-C₆)alkylsulfonyl radical.

22. Composition according to either of Claims 20 and 21, in which, in formula (VII) , R₂₀ is a C₁-C₄ alkyl radical that may be substituted with a hydroxyl or methoxy radical, and R is a hydrogen radical or a C₁-C₄ alkyl radical that may be optionally substituted with a hydroxyl or methoxy radical.

23. Composition according to any one of Claims 1 to 22, in which W₁ is chosen from 5-aminopyrazole, 5-hydroxypyrazole, pyrazolo[1,5-a]pyridine, pyrazolo[1,5-a]pyrimidine, triaminopyrimidine and 2,3-diamino-6-alkoxypyridine radicals.

24. Composition according to Claim 23, in which W₁ is chosen from 5-aminopyrazole and 5-hydroxypyrazole radicals.

25. Composition according to Claim 24, in which W₁ is chosen from 5-aminopyrazole and 5-hydroxypyrazole radicals with R₇ and R₁₁ chosen independently from a hydrogen atom; a linear or branched C₁-C₄ hydrocarbon-based chain that can form one or more 5- or 6-membered carbon-based rings and that may be saturated or unsaturated, the carbon atoms may be, independently of each other, substituted with one or more halogen atoms or hydroxyl or amino radicals; a C₁-C₂ alkoxy radical; an amino radical; a (di)(C₁-C₄)alkylamino radical.

26. Composition according to Claim 23, in which W₁ represents 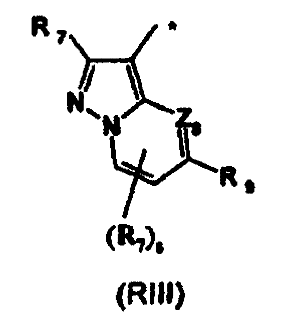 R₇, R₉ and Z₈ being as defined in the preceding claims.

27. Composition according to Claim 26, in which W₁ is such that Z₈ represents CR₁₂, then R₇, R₉ and R₁₂, which may be identical or different, are preferably chosen from:
• a hydrogen atom,
• an amino radical,
• a linear or branched C₁-C₈ hydrocarbon-based chain, which may form one or more 5- to 8-membered carbon-based rings, and which may be saturated or unsaturated, one or more carbon atoms of the carbon-based chain of which may be replaced with an oxygen, nitrogen or sulfur atom or with an SO₂ group, and the carbon atoms of which may be, independently of each other, substituted with one or more halogen atoms; the radicals not comprising a peroxide bond or diazo or nitroso radicals, and not being linked directly to the nitrogen atom via an oxygen, sulfur or nitrogen atom.

28. Composition according to Claim 27, in which the radicals R₇, R₉ and R₁₂ are chosen from a hydrogen atom; a linear or branched C₁-C₄ hydrocarbon-based chain that may be saturated or unsaturated, the carbon atoms may be, independently of each other, substituted with one or more halogen atoms or hydroxyl or amino radicals; a C₁-C₂ alkoxy radical; an amino radical; an optionally substituted (di)(C₁-C₄)alkylamino radical.

29. Composition according to Claim 26, in which W₁ is such that Z₈ represents N and R₇ and R₉ are chosen from a hydrogen atom; a linear or branched C₁-C₆ alkyl radical; a C₁-C₆ monohydroxyalkyl radical; a C₂-C₆ polyhydroxyalkyl radical; a C₁-C₆ aminoalkyl radical, a C₁-C₆ aminoalkyl radical in which the amine is mono- or disubstituted with a (C₁-C₆)alkyl or (C₁-C₆)alkylcarbonyl radical; a hydroxyl or amino radical, the amino possibly being substituted with one or more linear or branched C₁₋C₁₀ alkyl radicals, which can form one or more 5- to 6-membered carbon-based rings, and which may be saturated or unsaturated, the carbon atoms may be, independently of each other, substituted with one or more halogen atoms or hydroxyl or amino radicals; a C₁-C₂ alkoxy radical.

30. Composition according to Claim 26, in which R₇, R₉ and R₁₂ are chosen from hydrogen, a C₁-C₄ alkyl radical, an amino radical, a C₁-C₄ mono- or dialkylamino radical or a C₁-C₄ hydroxyalkyl radical; a C₁-C₄ alkoxy radical.

31. Composition according to any one of Claims 1 to 30, in which the compound of formula (I) is a cationic compound substituted with a cationic radical D-Z.

32. Composition according to any one of Claims 1 to 31, in which at least one of the radicals R₁ and R₂ is a cationic radical -D-Z.

33. Composition according to any one of Claims 1 to 31, in which R₁ and R₂ form the ring of formula (IV) in which R' is a cationic radical D1-Z.

34. Composition according to Claim 33, in which R' is chosen from an imidazolium and a trialkylammonium.

35. Composition according to Claim 1, in which Y is N+R' ₆R' ₇ , R'₆ and R'₇ being a C₁-C₄ alkyl radical.

36. Composition according to any one of the preceding claims, in which the compound of formula (I) represents 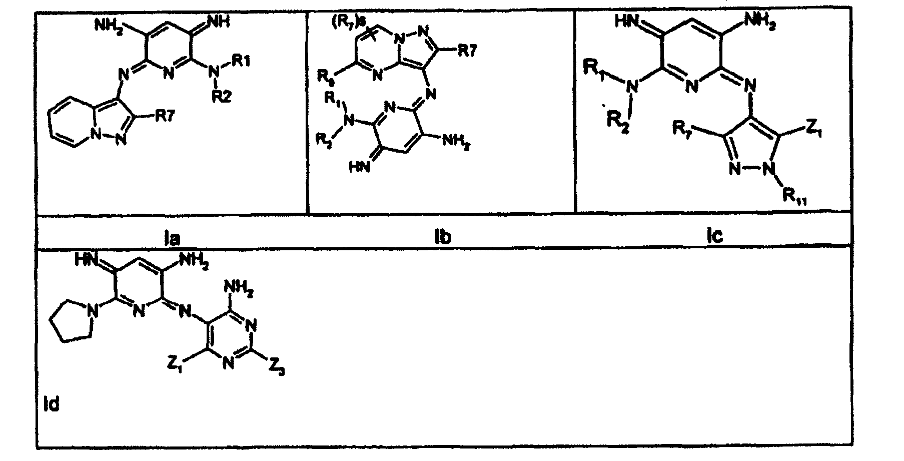 in which R₁, R₂, R₆, R₇, R₈, R₉, R₁₁, Z₁, Z₂ and Z₃ and s are as defined in the preceding claims.

37. Composition according to any one of Claims 1 to 36, in which the compound of formula (I) is chosen from

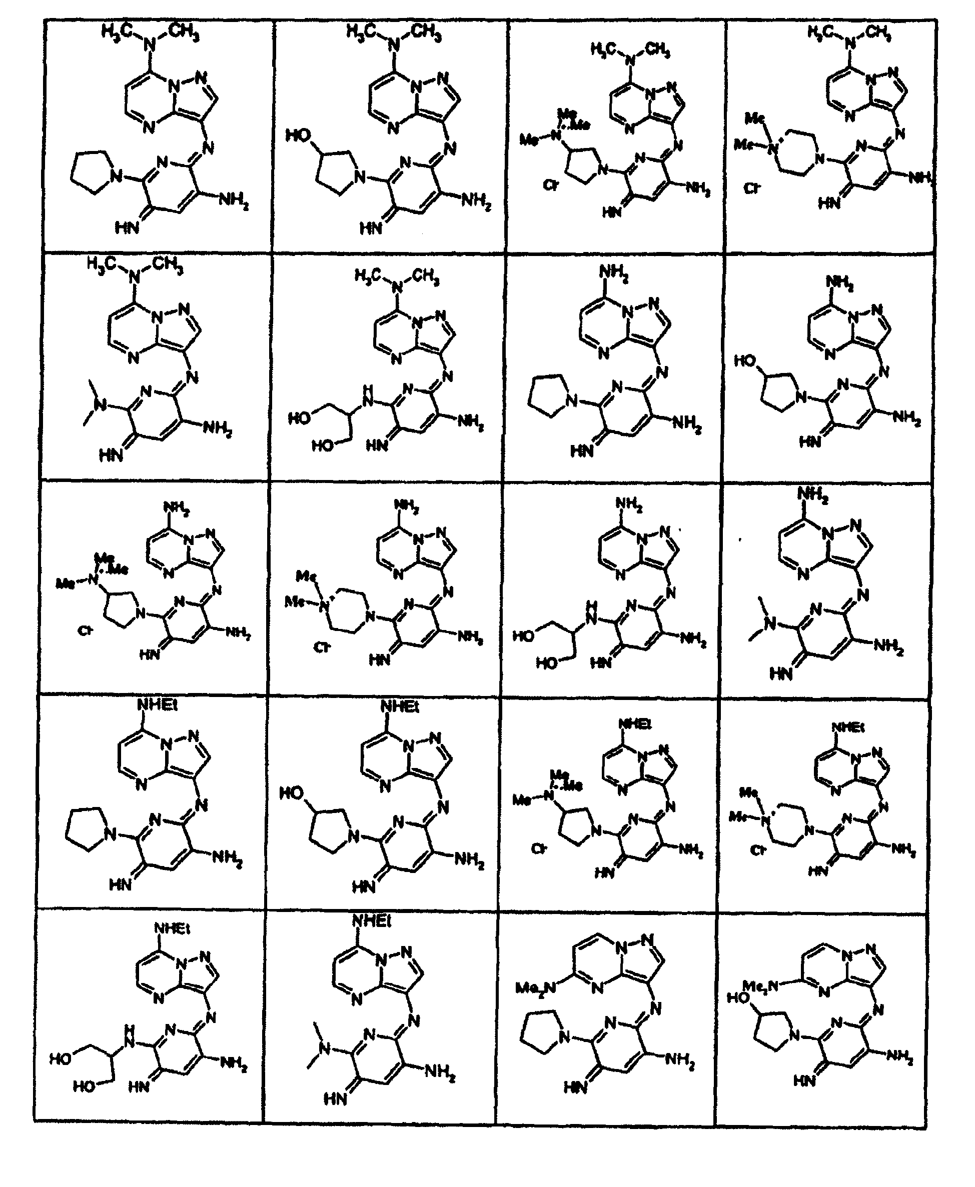

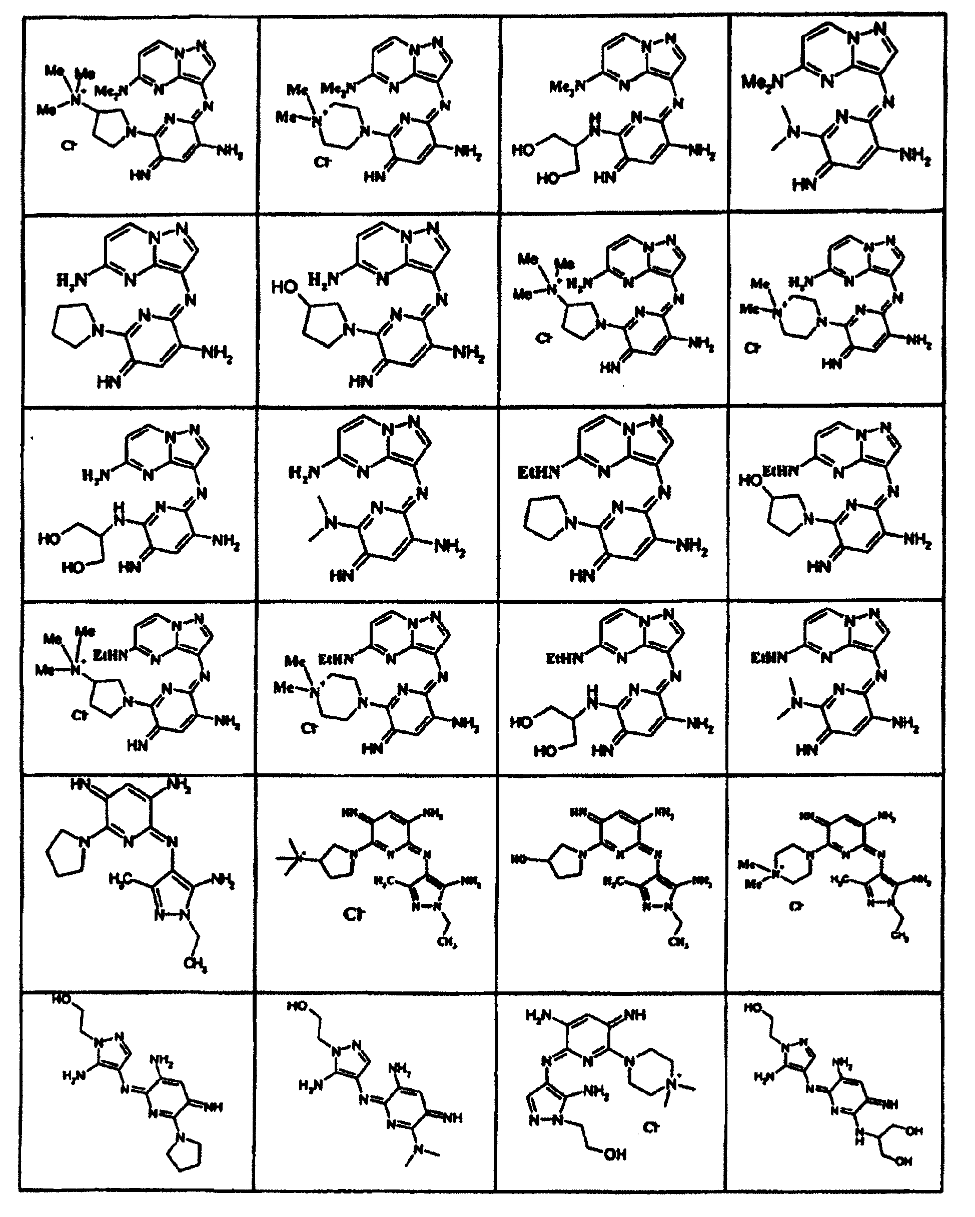

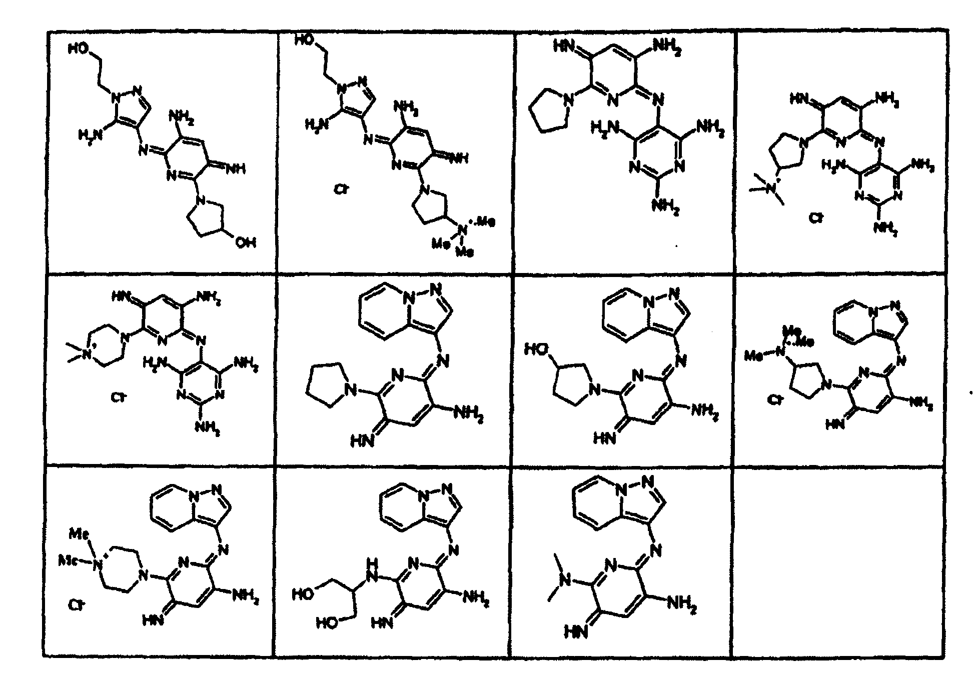

38. Composition according to any one of Claims 1 to 37, in which the amount of dye (I) is between 0.01% and 10% by weight.

39. Composition according to any one of Claims 1 to 38, also comprising an oxidation base chosen from paraphenylenediamines, bis(phenyl)alkylenediamines, para- aminophenols, ortho-aminophenols and heterocyclic bases, and the addition salts thereof with an acid.

40. Composition according to Claim 39, in which the oxidation base(s) is (are) present in an amount of between 0.001% and 10%.

41. Composition according to any one of Claims 1 to 40, comprising at least one coupler chosen from metaphenylenediamines, meta-aminophenols, meta-diphenols, naphthalene-based couplers and heterocyclic couplers, and the addition salts thereof with an acid.

42. Composition according to any one of Claims 1 to 41, also comprising an oxidizing agent.

43. Direct dye of formula (I) as defined in any one of Claims 1 to 37.

44. Process for dyeing keratin fibres, which comprises the application of the composition according to any one of Claims 1 to 42 for a time that is sufficient to obtain the desired coloration.

## Patentansprüche

1. Farbmittelzusammensetzung, die in einem geeigneten Medium eine Verbindung der folgenden Formel (I) enthält: 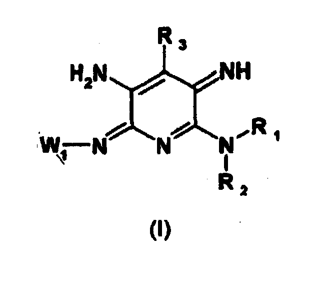 wobei in der Formel:
• R₃ bedeutet
- ein Wasserstoffatom,
- eine geradkettige oder verzweigte C₁₋₁₀-Kohlenwasserstoff kette, die einen oder mehrere 5- bis 8- gliedrige Kohlenstoffringe bilden kann, die gesättigt oder ungesättigt sein können, wobei ein oder mehrere Kohlenstoffatome der Kohlenstoffkette durch ein Sauerstoffatom, Stickstoffatom oder Schwefelatom oder durch eine Gruppe SO₂ ersetzt sein können und wobei die Kohlenstoffatome unabhängig voneinander mit einem oder mehreren Halogenatomen substituiert sein können; wobei R₃ weder eine Peroxidbindung, noch eine Diazogruppe, Nitrogruppe oder Nitrosogruppe aufweist und wobei R₃ nicht über ein Sauerstoffatom, Stickstoffatom oder Schwefelatom an den Pyridinring gebunden ist;
• R₁ und R₂ bedeuten unabhängig voneinander:
- ein Wasserstoffatom,
- eine geradkettige oder verzweigte C₁₋₁₀-Kohlenwasserstoff kette, die einen oder mehrere 5- bis 8- gliedrige Kohlenstoffringe bilden können, die gesättigt oder ungesättigt sein können, wobei ein oder mehrere Kohlenstoffatome der Kohlenstoffkette durch ein Sauerstoffatom, Stickstoffatom oder Schwefelatom oder durch eine Gruppe SO₂ ersetzt sein können und bei denen die Kohlenstoffatome unabhängig voneinander mit einem oder mehreren Halogenatomen substituiert sein können; wobei R₁ und R₂ weder eine Peroxidbindung noch eine Diazogruppe, Nitrogruppe oder Nitrosogruppe aufweisen und wobei R₁ und R₂ nicht über ein Sauerstoffatom, Schwefelatom oder Stickstoffatom an das Stickstoffatom gebunden sind,
- eine kationische Gruppe -D-Z, wobei Z eine Oniumgruppe ist und D eine geradkettige oder verzweigte C₁₋₁₄-Kohlenwasserstoffkette bedeutet, die einen oder mehrere 5- bis 8-gliedrige Kohlenstoffringe bilden kann, die gesättigt oder ungesättigt sein können, wobei ein oder mehrere Kohlenstoffatome der Kohlenstoffkette durch ein Sauerstoffatom, Stickstoffatom oder Schwefelatom oder durch eine Gruppe SO₂ ersetzt sein können; wobei die Verbindungsgruppe D nicht über ein Stickstoffatom, Sauerstoffatom oder Schwefelatom an das Stickstoffatom gebunden ist;
• Ri und R₂ bilden gemeinsam mit dem Stickstoffatom, an das sie gebunden sind, einen Ring der Formel (IV): 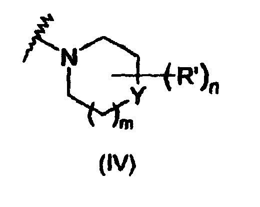
wobei in der Formel:
• R' bedeutet:
- ein Halogenatom;
- eine C₁₋₄-Alkylgruppe, die gegebenenfalls mit einer oder mehreren Gruppen substituiert ist, die unter den Gruppen Hydroxy, Carboxy, Alkoxycarbonyl(C₁₋₄), Alkyl(C₁₋₄)-amido (Alkyl(C₁₋₄)CONH-), Alkyl(C₁₋₄)carboxamido (Alkyl(C₁₋₄)NHCO-), Alkyl(C₁₋₄)sulfonyl (Alkyl(C₁₋₄)SO₂-), Alkoxy(C₁₋₄), Alkyl(C₁₋₄)sulfonamido (Alkyl(C₁₋₄)SO₂NH-), Alkyl(C₁₋₄)sulfamoyl (Alkyl(C₁₋₄)NHSO₂-) ausgewählt sind;
- NR'₃R'₄, wobei R'₃ und R'₄, die gleich oder verschieden sind, ein Wasserstoffatom; eine C₁₋₄-Alkylgmppe bedeuten, die gegebenenfalls mit einer oder mehreren Gruppen substituiert ist, die unter den Gruppen Hydroxy, C₁₋₄-Alkoxy, Amino, Mono- oder Dialkylamino, Alkyl(C₁₋₄)CO-, Alkyl(C₁₋₄)NHCO, Alkyl(C₁₋₄)SO₂ ausgewählt sind;
- eine Carboxygruppe;
- eine C₁₋₄-Alkoxycarbonylgruppe;
- eine Gruppe Alkyl(C₁₋₄)amido (Alkyl(C₁₋₄)CONH-);
- eine Gruppe Alkyl(C₁₋₄)sulfonyl (AlkylSO₂-);
- eine Gruppe Alkylsulfonamido (Alkyl(C₁₋₄)SO₂NH-);
- eine Hydroxygruppe;
- eine C₁₋₄-Alkoxygruppe;
- eine C₂₋₄-Hydroxyalkoxygruppe;
- eine Gruppe Alkyl(C₁₋₄)carboxamido (Alkyl(C₁₋₄)NHCO-);
- eine Gruppe Alkyl(C₁₋₄)sulfamoyl (Alkyl(C₁₋₄)-NH-SO₂-);
- eine C₁₋₄-Thioethergruppe;
- eine Sulfonsäuregruppe (SO₃H), die in Form des Salzes vorliegen kann;
- eine kationische Gruppe -D1-Z, bei der D1 eine kovalente Bindung oder D bedeutet,
- n 0 oder eine ganze Zahl von 1 bis 12 bedeutet,
- m 0 oder eine ganze Zahl von 1 bis 2 bedeutet,
- Y ein Kohlenstoffatom, ein Sauerstoffatom, eine Gruppe NR'₅ oder eine Gruppe N⁺R'₆R'₇ ist, wobei R'₅ die gleiche Bedeutung wie R'₃ hat; und R'₆ und R'₇ die gleiche Bedeutung wie R'₃ aufweisen und von einem Wasserstoffatom verschieden sind, mit der Maßgabe, dass die Gruppen R' voneinander verschieden sein können, wenn n > 1 ist,
• W₁ bedeutet eine aromatische heterocyclische Gruppe, die unter den folgenden Gruppen ausgewählt ist: 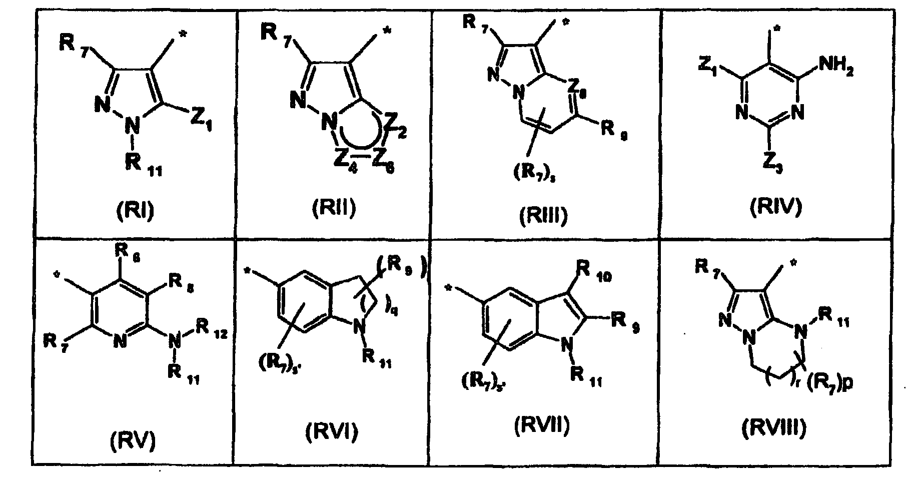
worin bedeuten
• s 0, 1 oder 2;
• s' 0, 1, 2 oder 3;
• Z₁ und Z₃ unabhängig voneinander eine Hydroxygruppe oder NR₁₁R₁₂,
• Z₂, Z₄ und Z₆ unabhängig voneinander ein Stickstoffatom, eine Gruppe CR₁₂ oder NR₁₁, mit der Maßgabe, dass mindestens eine dieser Gruppen eine Gruppe CR₁₂ bedeutet und nicht mehr als 3 Stickstoffatome enthalten sind,
• Z₈ bedeutet ein Stickstoffatom, eine Gruppe CR₁₂,
• R₆ und R₈ weisen unabhängig voneinander die gleichen Bedeutungen auf wie R₃,
• R₇, R₉, R₁₀, R₁₁ und R₁₂ bedeuten unabhängig voneinander:
- ein Wasserstoffatom,
- eine geradkettige oder verzweigte C₁₋₁₀-Kohlenwasserstoffkette, die einen oder mehrere 5- bis 8- gliedrige Kohlenstoffringe bilden kann, die gesättigt oder ungesättigt sein können, wobei ein oder mehrere Kohlenstoffatome der Kohlenstoffkette durch ein Sauerstoffatom, ein Stickstoffatom oder ein Schwefelatom oder durch eine Gruppe SO₂ ersetzt sein können und wobei die Kohlenstoffatome unabhängig voneinander mit einem oder mehreren Halogenatomen substituiert sein können; wobei die Gruppen R₇ und R₉ bis R₁₂ weder eine Peroxidbindung noch eine Diazogruppe oder Nitrosogruppe enthalten und wobei die Gruppen R₁₁ und R₁₂ nicht über ein Sauerstoffatom, Schwefelatom oder Stickstoffatom an das Stickstoffatom gebunden sind, mit der Maßgabe, dass die Gruppen R₇ und R₉ unabhängig voneinander so sein können:
- p kann Werte von 4 bis 8 annehmen,
- q kann Werte von 1 bis 3 annehmen,
- r kann Werte von 0 bis 2 annehmen,
- * bedeutet die Bindungsstelle von W1 in der Formel (I), mit der Maßgabe, dass die Verbindung der Formel (I) nicht mehr als eine kationische Gruppe D1-Z enthalten kann.

2. Zusammensetzung nach Anspruch 1, wobei die Verbindung (I) so vorliegt, dass R₃ unter einem Wasserstoffatom oder einer C₁₋₄-Alkylgruppe ausgewählt ist, die gegebenenfalls mit einer oder mehreren Gruppen substituiert ist, die unter den Gruppen Hydroxy, C₁₋₂-Alkoxy, Amino, (Mono)- oder (Di)alkylamino mit 1 bis 2 Kohlenstoffatomen ausgewählt sind.

3. Zusammensetzung nach Anspruch 1 oder 2, wobei die Verbindung (I) so vorliegt, dass R₃ unter einem Wasserstoffatom, Methyl, Ethyl oder 2-Hydroxyethyl ausgewählt ist.

4. Zusammensetzung nach Anspruch 1, 2 oder 3, wobei die Verbindung (I) so vorliegt, dass R₁ und R₂ unabhängig voneinander ausgewählt sind unter Wasserstoff, einer C₁₋₆-Alkylgruppe, die gegebenenfalls substituiert ist mit Hydroxy, Alkoxy, Amino, (Mono)- oder (Di)alkylamino(C₁₋₄), einer kationischen Gruppe -D-Z.

5. Zusammensetzung nach einem der Ansprüche 1 bis 4, wobei die Verbindung (I) so ist, dass R₁ oder R₂ unabhängig eine Gruppe -D-Z bedeutet, wobei Z unter Imidazolium, Trialkyl(C₁₋₄)ammonium, Pyridinium oder Piperidinium ausgewählt ist.

6. Zusammensetzung nach einem der Ansprüche 1 bis 5, wobei die Verbindung (I) so vorliegt, dass R₁ und R₂ unabhängig voneinander unter Wasserstoff, Methyl, Ethyl, Hydroxyethyl, Propyl, Propylimidazolium, Propyltrimethylammonium ausgewählt sind.

7. Zusammensetzung nach Anspruch 1, 2 oder 3, wobei die Verbindung (I) so ist, dass R₁ und R₂ mit dem Stickstoffatom, an das sie gebunden sind, einen 5- bis 8-gliedrigen Heterocyclus bilden, der unter den Heterocyclen Pyrrolidin, Piperidin, Homopiperidin, Piperazin, Homopiperazin und Diazepan ausgewählt ist.

8. Zusammensetzung nach Anspruch 7, wobei die Verbindung (I) so vorliegt, dass R₁ und R₂ einen Heterocyclus bilden, der unter Pyrrolidin, 3-Hydroxypyrrolidin, 3-Aminopyrrolidin, 3-N,N-Dimethylaminopyrrolidin, 3-Acetamidopyrrolidin, 3-(Methylsulfonylamino)-pyrrolidin, Prolin, 3-Hydroxyprolin, 3-(N-Methyl-imidazolium)-pyrrolidin, 3-(Trialkyl(C₁₋₄)ammonium)-pyrrolidin, Piperidin, Hydroxypiperidin, Homopiperidin, Diazepan, N-Methylhomopiperazin, N-β-Hydroxyethylhomopiperazin und deren Additionssalzen ausgewählt ist.

9. Zusammensetzung nach Anspruch 7, wobei die Verbindung der Formel (I) so ist, dass R₁ und R₂ mit dem Stickstoffatom, an das sie gebunden sind, einen gegebenenfalls substituierten Pyrrolidinring bilden.

10. Zusammensetzung nach einem der Ansprüche 1 bis 9, wobei die Verbindung (I) so vorliegt, dass die kationische Gruppe -D-Z der folgenden Formel (V) entspricht: 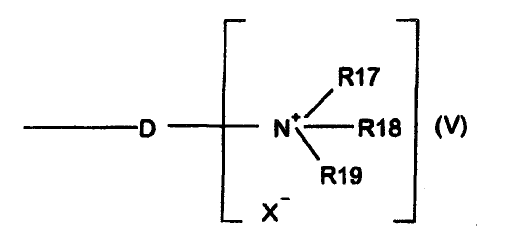 wobei in der Formel
• D die in Anspruch 1 angegebenen Bedeutungen hat,
• R₁₇, R₁₈ und R₁₉ unabhängig voneinander bedeuten: C₁₋₁₅-Alkyl; C₁₋₆-Monohydroxyalkyl; C₂₋₆-Polyhydroxyalkyl; Alkoxy(C₁₋₆)alkyl(C₁₋₆); Aryl; Benzyl; Amidoalkyl(C₁₋₆); Trialkyl(C₁₋₆)-silanalkyl(C₁₋₆); Aminoalkyl(C₁₋₆); Aminoalkyl(C₁₋₆), wobei die Aminogruppe mono- oder disubstituiert ist mit C₁₋₄-Alkyl, Alkyl(C₁₋₆)carbonyl, Acylamino oder Alkyl(C₁₋₆)sulfonyl;
• R₁₇, R₁₈ und R₁₉ gemeinsam paarweise mit dem Stickstoffatom, an das sie gebunden sind, einen gesättigten, 5-, 6- oder 7-gliedrigen Kohlenstoffring bilden können, der ein oder mehrere Heteroatome enthalten kann, wobei der kationische Ring substituiert sein kann mit einem Halogenatom, einer Hydroxygruppe, einer C₁₋₆-Alkylgruppe, einer C₁₋₆-Monohydroxy-alkylgruppe, einer C₂₋₆-Polyhydroxyalkylgruppe, C₁₋₆-Alkoxy, Trialkyl(C₁₋₆)silanalkyl(C₁₋₆), Amido, Carboxy, Alkyl(C₁₋₆)-carbonyl, Thio, Thioalkyl(C₁₋₆), Alkyl(C₁₋₆)thio, Amino, einer Aminogruppe, die mono- oder disubstituiert ist mit Alkyl(C₁₋₆), Alkyl(C₁₋₆)carbonyl, Acylamino oder Alkyl(C₁₋₆)sulfonyl;
• eine der Gruppen R₁₇, R₁₈ und R₁₉ an eines der Kohlenstoff atome oder Stickstoffatome der Verbindungsgruppe D gebunden sein kann, sodass ein 5- bis 7-gliedriger Ring gebildet wird, und
• X- ein Gegenion bedeutet.

11. Zusammensetzung nach Anspruch 10, wobei R₁₇, R₁₈ und R₁₉ getrennt voneinander ausgewählt sind unter C₁₋₆-Alkyl, C₁₋₄-Monohydroxyalkyl, C₂₋₄-Polyhydroxyalkyl, Alkoxy(C₂₋₆)alkyl(C₁₋₄), Amidoalkyl(C₂₋₆), Trialkyl(C₁₋₆)silanalkyl(C₁₋₆).

12. Zusammensetzung nach Anspruch 10, wobei R₁₇ zusammen mit R₁₈ einen Ring Pyrrolidinium, Piperidinium, Homopiperidinium, Piperazinium, Homopiperazinium oder Morpholinium bildet, wobei R₁₉ dann ausgewählt ist unter Alkyl(C₁₋₆); Monohydroxyalkyl(C₁₋₆); Polyhydroxyalkyl(C₂₋₆); Aminoalkyl(C₂₋₆), einer Aminoalkylgruppe, die mono- oder disubstituiert ist mit Alkyl(C₁₋₆), Alkyl(C₁₋₆)carbonyl; Carbamoylalkyl(C₂₋₆); Trialkyl(C₁₋₆)silanalkyl(C₁₋₆); Alkyl(C₁₋₆)carboxyalkyl(C₁₋₆); Alkyl(C₁₋₆)carbonylalkyl(C₂₋₆); N-Alkyl(C₁₋₆)carbamoylalkyl(C₂₋₆).

13. Zusammensetzung nach Anspruch 10, wobei die Gruppen R₁₇, R₁₈ und R₁₉ Alkylgruppen sind.

14. Zusammensetzung nach einem der Ansprüche 10 bis 13, wobei D eine C₁₋₆-Alkylenkette bedeutet, die substituiert sein kann.

15. Zusammensetzung nach einem der Ansprüche 1 bis 9, wobei die kationische Gruppe -D-Z der Formel (VI) entspricht 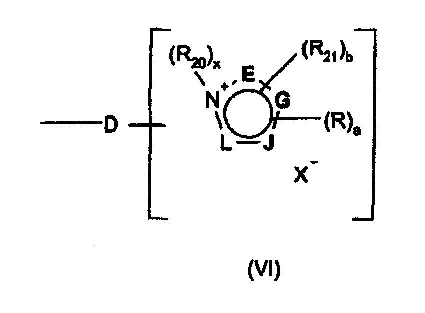 wobei in der Formel
• D die in Anspruch 1 angegebenen Bedeutungen hat,
• die Ringbestandteile E, G, J und L, die gleich oder verschieden sind, ein Kohlenstoffatom, ein Sauerstoffatom, ein Schwefelatom oder ein Stickstoffatom bedeuten, sodass ein Pyrazoliumring, Imidazoliumring, Triazoliumring, Oxazoliumring, I-sooxazoliumring, Thiazoliumring, Isothiazoliumring gebildet wird,
• a eine ganze Zahl im Bereich von 1 bis einschließlich 3 ist;
• b 0 oder 1 bedeutet;
• die Gruppen R, die gleich oder verschieden sind, bedeuten: ein Wasserstoffatom, ein Halogenatom, eine C₁₋₆-Alkylgruppe, eine C₁₋₆-Monohydroxyalkylgruppe, eine C₂₋₆-Polyhydroxyalkylgruppe, eine C₁₋₆-Alkoxygruppe, eine Amidogruppe, eine Carboxygruppe, eine C₁₋₆-Alkylcarbonylgruppe, eine C₁₋₆-Thioalkylgruppe, eine Alkyl(C₁₋₆)thiogruppe, eine Aminogruppe, die mit Alkyl(C₁₋₆), Alkyl(C₁₋₆)carbonyl, Alkyl(C₁₋₆)sulfonyl disubstituiert ist, Benzyl, eine Phenylgruppe, die gegebenenfalls mit einer oder mehreren Gruppen substituiert ist, die unter Methyl, Hydroxy, Amino, Methoxy ausgewählt sind, mit der Maßgabe, dass die Gruppen R von einem Kohlenstoffatom getragen werden,
• R₂₁ bedeutet: C₁₋₆-Alkyl; C₁₋₆-Monohydroxyalkyl, C₂₋₆-Polyhydroxyalkyl, Trialkyl(C₁₋₆)silanalkyl(C₁₋₆), Alkoxy(C₁₋₆)alkyl-(C₁₋₆), Carbamoylalkyl(C₂₋₆), Alkyl(C₁₋₆)carboxyalkyl(C₁₋₆), Benzyl, mit der Maßgabe, dass die Gruppe R₂₁ von einem Stickstoffatom getragen wird,
• R₂₀ bedeutet: C₁₋₆-Alkyl; C₁₋₆-Monohydroxyalkyl; C₂₋₆-Polyhydroxyalkyl; Benzyl; C₁₋₆-Aminoalkyl; C₁₋₆-Aminoalkyl, bei der die Aminogruppe mono- oder disubstituiert ist mit Alkyl(C₁₋₆), Alkyl(C₁₋₆)carbonyl, Acylamino oder Alkyl(C₁₋₆)sulfonyl; Carboxyalkyl(C₁₋₆); Carbamoylalkyl(C₁₋₆); Trifluoralkyl(C₂₋₆); Trialkyl(C₁₋₆)silanalkyl(C₁₋₆); Sulfonamidoalkyl(C₂₋₆); Alkyl(C₁₋₆)-carboxyalkyl(C₁₋₆); Alkyl(C₁₋₆)sulfinylalkyl(C₂₋₆); Alkyl(C₁₋₆)sulfonylalkyl(C₂₋₆); Alkyl(C₁₋₆)carbonylalkyl(C₂₋₆); N-Alkyl(C₁₋₆)carbamoylalkyl(C₂₋₆); N-Alkyl(C₁₋₆)sulfonamidoalkyl(C₂₋₆);
• x bedeutet 0 oder 1;
- wenn x = 0, ist die Verbindungsgruppe D an das Ammoniumatom gebunden,
- wenn x = 1, ist die Verbindungsgruppe D an einen Ringbestandteil E, G, J oder L gebunden;
• X- ist ein Gegenion.

16. Zusammensetzung nach Anspruch 15, wobei die Ringbestandteile einen Imidazoliumring, Pyrazoliumring, Oxazoliumring, Thiazoliumring und Triazoliumring bilden.

17. Zusammensetzung nach Anspruch 15 oder 16, wobei die Formel (VI) so ist, dass x 0 ist und D eine C₁₋₄-Kohlenwasserstoffkette bedeutet, die substituiert sein kann.

18. Zusammensetzung nach einem der Ansprüche 1 bis 9, wobei die kationische Gruppe -D-Z der Formel (VII) entspricht 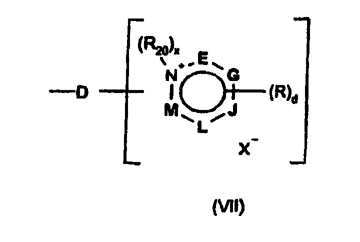 wobei in der Formel:
• D die in Anspruch 1 angegebene Bedeutung hat,
• R und R₂₀ die in Anspruch 17 angegebene Bedeutung aufweisen,
• die Ringbestandteile E, G, J, L und M, die gleich oder verschieden sind, ein Kohlenstoffatom oder Stickstoffatom bedeuten und einen Ring bilden, der unter Pyridinium, Pyrimidinium, Pyrazinium und Pyridazinium ausgewählt ist,
• d ist eine ganze Zahl von 1 bis einschließlich 5;
• x bedeutet 0 oder 1
- wenn x = 0, ist die Verbindungsgruppe D an das Ammoniumatom gebunden,
- wenn x = 1, ist die Verbindungsgruppe D an einen Ringbestandteil E, G, J, L oder M gebunden,
• X- ein Gegenion ist.

19. Zusammensetzung nach Anspruch 18, wobei in der Formel (VII) die Ringbestandteile E, G, J, L und M mit dem quaternisierten Stickstoffatom des Rings einen Ring bilden, der unter den Ringen Pyridinium, Pyrimidinium, Pyrazinium und Pyridazinium ausgewählt ist.

20. Zusammensetzung nach einem der Ansprüche 18 oder 19, wobei in der Formel (VII) x 0 ist und R ausgewählt ist unter einem Wasserstoffatom, C₁₋₆-Alkyl, C₁₋₆-Monohydroxyalkyl, C₂₋₆-Polyhydroxyalkyl, C₁₋₆-Alkoxy, C₁₋₆-Alkylcarbonyl, einer Aminogruppe, die disubstituiert ist mit Alkyl(C₁₋₆), Alkyl(C₁₋₆)carbonyl, Alkyl(C₁₋₆)sulfonyl; mit der Maßgabe, dass die Gruppen R von einem Kohlenstoffatom getragen werden.

21. Zusammensetzung nach einem der Ansprüche 18 oder 19, wobei in der Formel (VII) x 1 ist und R₂₀ ausgewählt ist unter C₁₋₆-Alkyl; C₁₋₆-Monohydroxyalkyl; C₂₋₆-Polyhydroxyalkyl; C₂₋₆-Aminoalkyl, einer C₂₋₆-Aminoalkylgruppe, bei der die Aminogruppe mono- oder disubstituiert ist mit Alkyl(C₁₋₆), Alkyl(C₁₋₆)carbonyl, Acylamino oder Alkyl(C₁₋₆)sulfonyl; Carbamoylalkyl(C₁₋₆); Alkyl(C₁₋₆)carbonylalkyl(C₁₋₆); N-Alkyl(C₁₋₆)carbamoylalkyl(C₁₋₆); R ist ausgewählt unter C₁₋₆-Alkyl, C₁₋₆-Monohydroxyalkyl, C₂₋₆-Polyhydroxyalkyl, C₁₋₆-Alkoxy, C₁₋₆-Alkylcarbonyl, einer Aminogruppe, die disubstituiert ist mit Alkyl(C₁₋₆), Alkyl(C₁₋₆)carbonyl, Alkyl(C₁₋₆)sulfonyl.

22. Zusammensetzung nach einem der Ansprüche 20 oder 21, wobei in der Formel (VII) R₂₀ eine C₁₋₄-Alkylgruppe ist, die mit einer Hydroxygruppe oder einer Methoxygruppe substituiert sein kann, und R ein Wasserstoffatom oder eine C₁₋₄-Alkylgruppe bedeutet, die gegebenenfalls mit einer Hydroxygruppe oder Methoxygruppe substituiert sein kann.

23. Zusammensetzung nach einem der Ansprüche 1 bis 22, wobei W₁ ausgewählt ist unter 5-Aminopyrazol, 5-Hydroxypyrazol, Pyrazolo-[1,5-a]-pyridin, Pyrazolo-[1,5-a]-pyrimidin, Triaminopyrimidin und 2,3-Diamino-6-alkoxypyridin.

24. Zusammensetzung nach Anspruch 23, wobei W 1 unter 5-Aminopyrazol und 5-Hydroxypyrazol ausgewählt ist.

25. Zusammensetzung nach Anspruch 24, wobei W 1 unter den Gruppen 5-Aminopyrazol und 5-Hydroxypyrazol ausgewählt ist, wobei R₇ und R₁₁ unabhängig voneinander ausgewählt sind unter einem Wasserstoffatom; einer geradkettigen oder verzweigten C₁₋₄-Kohlenwasserstoffkette, die einen oder mehrere 5- bis 6-gliedrigen Kohlenstoffringe bilden kann, die gesättigt oder ungesättigt sein können, wobei die Kohlenstoffatome unabhängig voneinander mit einem oder mehreren Halogenatomen oder Hydroxygruppen, Aminogruppen substituiert sein können; C₁₋₂-Alkoxy; Amino; (Di)alkyl(C₁₋₄)amino.

26. Zusammensetzung nach Anspruch 23, wobei W 1 bedeutet: 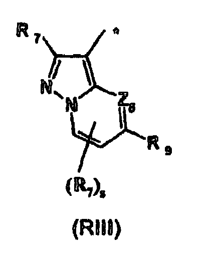 wobei R₇, R₉ und Z₈ die in den vorhergehenden Ansprüchen angegebenen Bedeutungen aufweisen.

27. Zusammensetzung nach Anspruch 26, wobei W1 so ist, dass Z₈ CR₁₂ bedeutet, wobei R₇, R₉ und R₁₂, die gleich oder verschieden sind, vorzugsweise ausgewählt sind unter:
• einem Wasserstoffatom,
• einer Aminogruppe,
• einer geradkettigen oder verzweigten C₁₋₈-Kohlenwasserstoffkette, die einen oder mehrere 5- bis 8-gliedrige Kohlenstoff ringe bilden kann, die gesättigt oder ungesättigt sein können, wobei ein oder mehrere Kohlenstoffatome der Kohlenstoffkette durch ein Sauerstoffatom, Stickstoffatom oder Schwefelatom oder durch eine Gruppe SO₂ ersetzt sein können und wobei die Kohlenstoffatome unabhängig voneinander mit einem oder mehreren Halogenatomen substituiert sein können; wobei die Gruppen weder eine Peroxidbindung noch Diazogruppen oder Nitrosogruppen enthalten und nicht über ein Sauerstoffatom, Schwefelatom oder Stickstoffatom direkt an das Stickstoffatom gebunden sind.

28. Zusammensetzung nach Anspruch 27, wobei die Gruppen R₇, R₉ und R₁₂ ausgewählt sind unter einem Wasserstoffatom; einer geradkettigen oder verzweigten C₁₋₄-Kohlenwasserstoffkette, wobei sie gesättigt oder ungesättigt sein können und wobei die Kohlenstoffatome unabhängig voneinander substituiert sein können mit einem oder mehreren Halogenatomen oder einer oder mehreren Hydroxygruppen, Aminogruppen; C₁₋₂-Alkoxy; Amino; Dialkyl(C₁₋₄)amino, wobei diese Gruppe gegebenenfalls substituiert ist.

29. Zusammensetzung nach Anspruch 26, wobei W1 so ist, dass Z₈ N bedeutet und R₇ und R₉ ausgewählt sind unter einem Wasserstoffatom; einer geradkettigen oder verzweigten C₁₋₆-Alkylgruppe; C₁₋₆-Monohydroxyalkyl; C₂₋₆-Polyhydroxyalkyl; C₁₋₆-Aminoalkyl, einer C₁₋₆-Aminoalkylgruppe, bei der die Aminogruppe mono- oder disubstituiert ist mit Alkyl(C₁₋₆), Alkyl(C₁₋₆)carbonyl; Hydroxy oder Amino, wobei die Aminogruppe gegebenenfalls mit einer oder mehreren geradkettigen oder verzweigten C₁₋₁₀-Alkylgruppen substituiert sein kann, die einen oder mehrere 5- bis 6-gliedrige Kohlenstoffringe bilden können, die gesättigt oder ungesättigt sein können, wobei die Kohlenstoffatome unabhängig voneinander mit einem oder mehreren Halogenatomen oder einer oder mehreren Hydroxygruppen, Aminogruppen substituiert sein können; C₁₋₂-Alkoxy.

30. Zusammensetzung nach Anspruch 26, wobei R₇, R₉ und R₁₂ ausgewählt sind unter Wasserstoff, C₁₋₄-Alkyl, Amino, Mono- oder Dialkylamino(C₁-C₄), C₁₋₄-Hydroxyalkyl; C₁₋₄-Alkoxy.

31. Zusammensetzung nach einem der Ansprüche 1 bis 30, wobei die Verbindung der Formel (I) eine kationische Verbindung ist, die mit einer kationischen Gruppe -D-Z substituiert ist.

32. Zusammensetzung nach einem der Ansprüche 1 bis 31, bei der mindestens eine der Gruppen R₁ und R₂ eine kationische Gruppe -D-Z ist.

33. Zusammensetzung nach einem der Ansprüche 1 bis 31, wobei R₁ und R₂ den Ring der Formel (IV) bilden, wobei R' eine kationische Gruppe D1-Z ist.

34. Zusammensetzung nach Anspruch 33, wobei R' ausgewählt ist unter Imidazolium oder Trialkylammonium.

35. Zusammensetzung nach Anspruch 1, wobei Y N⁺R'₆R'₇ bedeutet, wobei R'₆ und R'₇ C₁₋₄-Alkyl bedeuten.

36. Zusammensetzung nach einem der vorhergehenden Ansprüche, wobei die Verbindung der Formel (I) bedeutet: 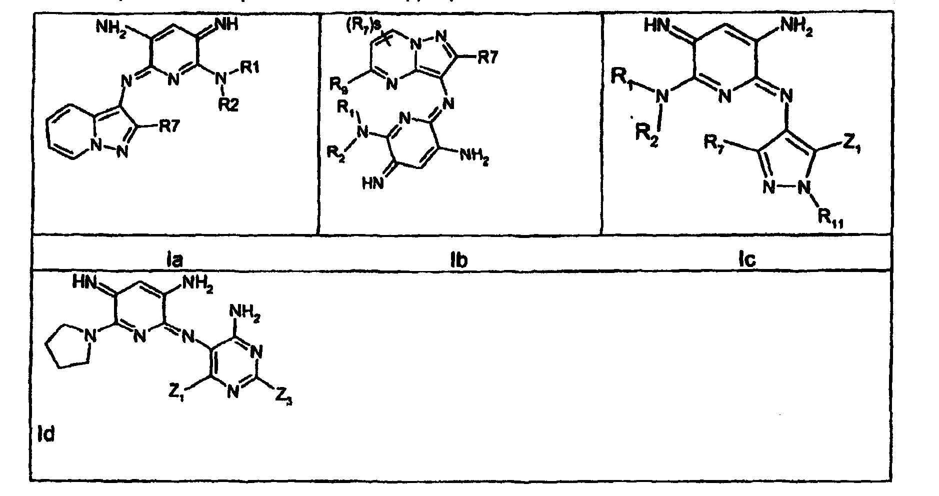 wobei R₁, R₂, R₆, R₇, R₈, R₉, R₁₁, Z₁, Z₂, Z₃ und s die in den vorhergehenden Ansprüchen angegebenen Bedeutungen aufweisen.

37. Zusammensetzung nach einem der Ansprüche 1 bis 36, wobei die Verbindung der Formel (I) ausgewählt ist unter

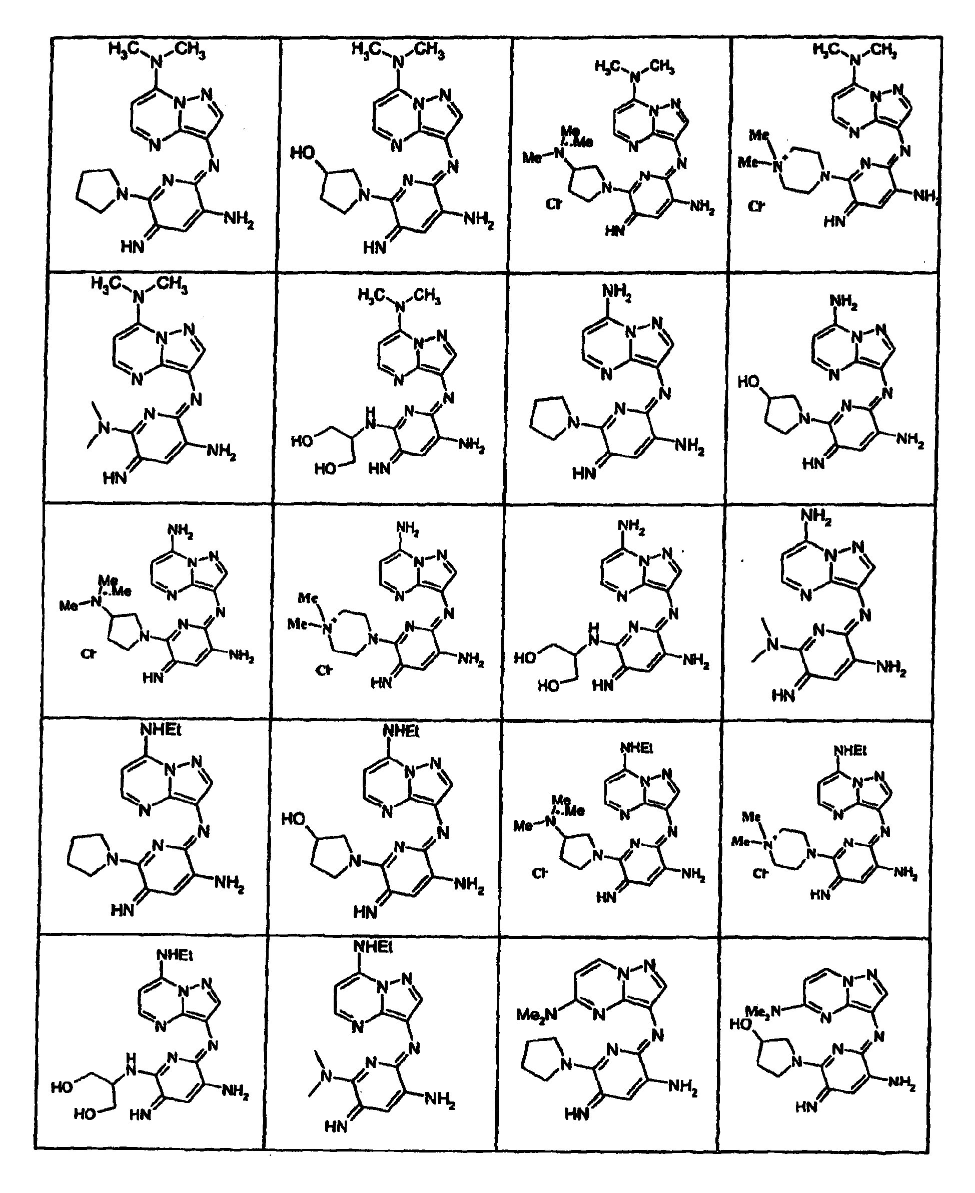

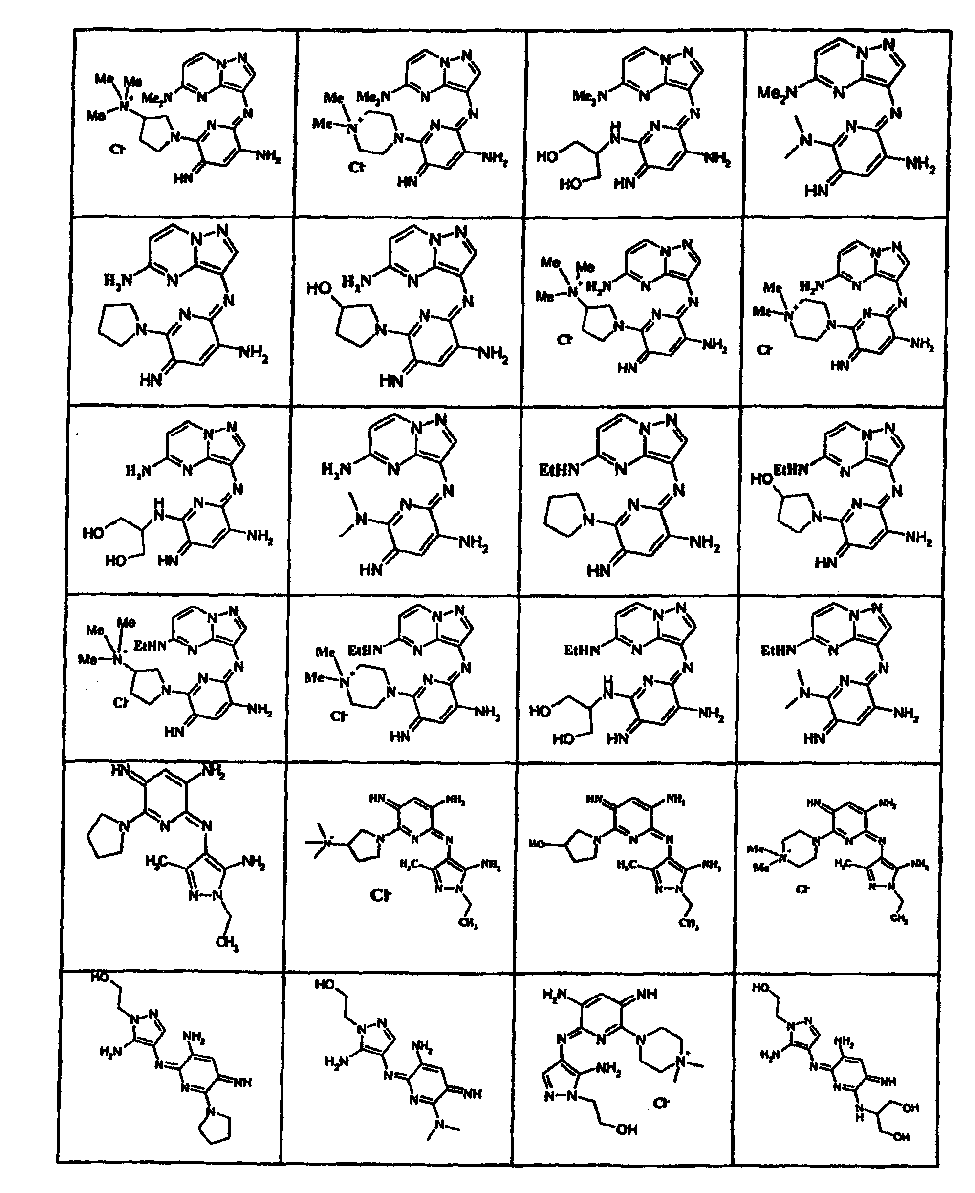

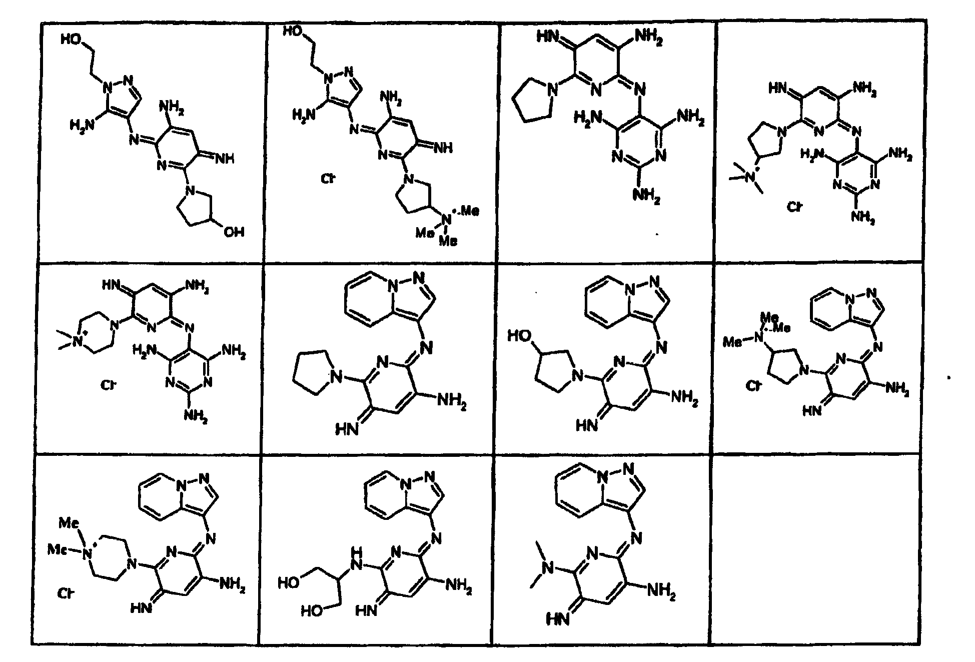

38. Zusammensetzung nach einem der Ansprüche 1 bis 37, wobei der Mengenanteil des Farbstoffs (I) im Bereich von 0,01 bis 10 Gew.-% liegt.

39. Zusammensetzung nach einem der Ansprüche 1 bis 38, die ferner eine Oxidationsbase enthält, die unter den p-Phenylendiaminen, Bisphenylalkylendiaminen, p-Aminophenolen, o-Aminophenolen, heterocyclischen Basen und deren Additionssalzen mit einer Säure ausgewählt ist.

40. Zusammensetzung nach Anspruch 39, wobei die Oxidationsbase(n) in einer Menge von 0,001 bis 10 % enthalten sind.

41. Zusammensetzung nach einem der Ansprüche 1 bis 40, die mindestens einen Kuppler enthält, der unter den m-Phenylendiaminen, m-Aminophenolen, *m*-Dihydroxybenzolen, Naphthalinkupplern und heterocyclischen Kupplern und deren Additionssalzen mit einer Säure ausgewählt ist.

42. Zusammensetzung nach einem der Ansprüche 1 bis 41, die ferner ein Oxidationsmittel enthält.

43. Direktfarbstoff der Formel (I) nach einem der Ansprüche 1 bis 37.

44. Verfahren zum Färben von Keratinfasern, das das Aufbringen der Zusammensetzung nach einem der Ansprüche 1 bis 42 während einer Zeitspanne umfasst, die ausreichend ist, um die gewünschte Färbung zu bilden.
